(19) Europäisches Patentamt
European Patent Office
Office européen des brevets

(11) Publication number: **0 220 840**
**A2**

# (12) EUROPEAN PATENT APPLICATION

(21) Application number: **86307457.1**

(22) Date of filing: **29.09.86**

(51) Int. Cl.4: **C07C 127/22**, C07D 339/06, C07D 317/04, C07D 317/28, C07D 339/08, C07D 319/06, A01N 47/34

(30) Priority: **30.09.85 US 781382**
**11.08.86 US 895364**

(43) Date of publication of application: **06.05.87 Bulletin 87/19**

(84) Designated Contracting States: **AT BE CH DE FR GB IT LI LU NL SE**

(71) Applicant: **UNION CARBIDE CORPORATION**
**39 Old Ridgebury Road**
**Danbury Connecticut 06817(US)**

(72) Inventor: **Caruso, Andrew James**
**275 Lakeside Drive Durham**
**North Carolina 27707(US)**

(74) Representative: **Baverstock, Michael George Douglas et al**
**BOULT, WADE & TENNANT 27 Furnival Street**
**London, EC4A 1PQ(GB)**

(54) **Pesticidal 1-(4-aryloxyphenyl)-3-benzoyl urea compounds, processes for their preparation, and pesticidal compositions and methods of controlling pests employing said compounds.**

(57) Novel 1-(4-aryloxyphenyl)-3-benzoyl urea compounds are described together with methods for their preparation, and the use of said compounds as the active toxicant in pesticidal compositions, and in methods of controlling pests.

EP 0 220 840 A2

# PESTICIDAL 1-(4-ARYLOXYPHENYL)-3-BENZOYL UREA COMPOUNDS; PROCESSES FOR THEIR PREPARATION; AND PESTICIDAL COMPOSITIONS AND METHODS OF CONTROLLING PESTS EMPLOYING SAID COMPOUNDS

This invention relates to novel 1-(4-aryloxyphenyl)-3-benzoyl urea compounds which are useful as the active toxicant in pesticidal compositions. This invention also relates to a method for the preparation of the novel 1-(4-aryloxyphenyl)-3-benzoyl urea compounds. This invention further relates to pesticidal compositions and to a method for their use.

In recent years a variety of benzoyl urea compounds have been reported in the literature as having pesticidal activity. For example, pyridyloxyphenyl benzoyl ureas and their use as insecticides have been disclosed in U.S. Patent 4,310,530 issued January 12, 1982, and U.S. Patent 4,344,951 issued August 17, 1982. Also, pyridyloxyphenyl benzoyl urea compounds and their use as insecticides have been disclosed in Japanese Patent Application 5 7099 569 published June 21, 1982, Japanese Patent Application 5 7109 768 published July 8, 1982, Japanese Patent Application 5 7144 258 published September 6, 1982, Japanese Patent Application 5 7114 259 published September 6, 1982, and Japanese Patent Application 5 7145 861 published September 9, 1982. Phenoxyphenyl benzoyl urea compounds have been disclosed in Japanese Patent Application 5 8035 163 published March 1, 1983 and Japanese Patent Application 5 8039 657 published March 8, 1983.

Accordingly, one or more of the following objects will be achieved by the practice of this invention. It is an object of this invention is to provide novel 1-(4-aryloxyphenyl)-3-benzoyl urea compounds. Another object of this invention is to provide certain 1-(4-aryloxyphenyl)-3-benzoyl urea compounds which exhibit excellent insecticidal activity. A still further object of this invention is to provide novel benzoyl urea compounds, such as 1-[3-chloro-4-(2,4-dichlorophenoxy)-5-dimethoxymethyl-2-methylphenyl]-3-(2,6-difluorobenzoyl) urea, 1-[3-chloro-4-(2,4-dichlorophenoxy)-5-formyl-2-methylphenyl]-3-(2,6-difluorobenzoyl) urea, 1-[3-chloro-4-(2,4-dichlorophenoxy)-5-hydroxymethyl-2-methylphenyl]-3-(2,6-difluorobenzoyl) urea, 1-[5-carboxy-3-chloro-4-(2,4-dimethylphenoxy)-2-methylphenyl]-3-(2,6-difluorobenzoyl) urea, 1-[3-chloro-4-(2,4-dimethylphenoxy)-5-(2-methoxycarbonyl-1-ethenyl)-2-methylphenyl]-3-(2,6-difluorobenzoyl) urea, etc. Another object is to provide processes for the preparation of the novel benzoyl urea compounds. A further object is to provide novel pesticidal compositions containing the novel benzoyl urea compounds as the active toxicant. Another object of the invention is to provide a method for controlling pests by the application of the novel pesticidal compositions. These and other objects will readily become apparent to those skilled in the art in the light of the teachings herein set forth.

In its broad aspect the invention relates to novel 1-(4-aryloxyphenyl)-3-benzoyl urea compounds, pesticidal compositions containing the same, and processes for their preparation and use. The benzoyl urea compounds of this invention can be represented by the following formula:

FORMULA (1)

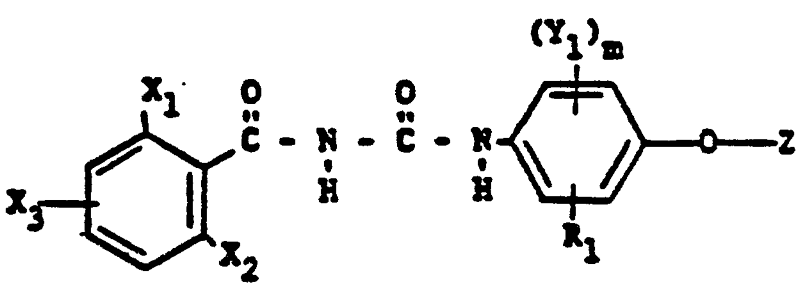

wherein:

$X_1$ represents halogen;

$X_2$ and $X_3$ independently represent hydrogen or halogen;

$Y_1$ independently represents halogen, alkyl, alkoxy, nitro or cyano;

m is a value of from 0 to 2;

$R_1$ represents an acyclic or cyclic acetal group, an acyclic or cyclic dithioacetal group, an acyclic or cyclic hemithioacetal group, aldehyde group, carboxylic acid group or ester derivative thereof, hydroxyalkyl group,

alkoxyalkyl group, acyloxyalkyl group, alkenyl group or alkylcarbonyl group; with the proviso that when $R_1$ is a carboxylic acid group or ester derivative thereof, then m is a value of 2, and with the further proviso that $R_1$ is situated at other than the 2-or 6-position; and

Z represents (i) a substituted or unsubstituted, saturated or unsaturated monocyclic ring system or (ii) a substituted or unsubstituted, saturated or unsaturated bicyclic fused ring system wherein at least one ring is a six-membered unsaturated carbocyclic ring and the second ring is a five-or six-membered carbocyclic ring or a five-or six-membered heterocyclic ring which can contain in any combination a carbonyl group or one or two oxygen or sulfur atoms.

As indicated above, the invention relates to novel 1-(4-aryloxyphenyl)-3-benzoyl urea compounds, pesticidal compositions containing the same, and processes for their preparation and use.

Preferred benzoyl urea compounds within the broad generic Formula (1) are those having the formula:

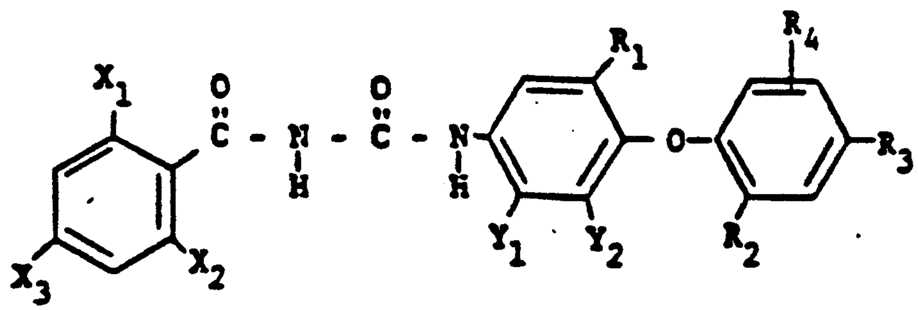

wherein $X_1$, $X_2$, $X_3$, $R_1$ and $Y_1$ are as indicated above, $Y_2$ independently has the meaning given for $Y_1$, $R_2$ and $R_3$ are independently hydrogen, halogen, alkyl, polyhaloalkyl, nitro or cyano, and $R_4$ is hydrogen, halogen, alkyl or polyhaloalkyl.

Particularly preferred benzoyl urea compounds are those of the formulas:

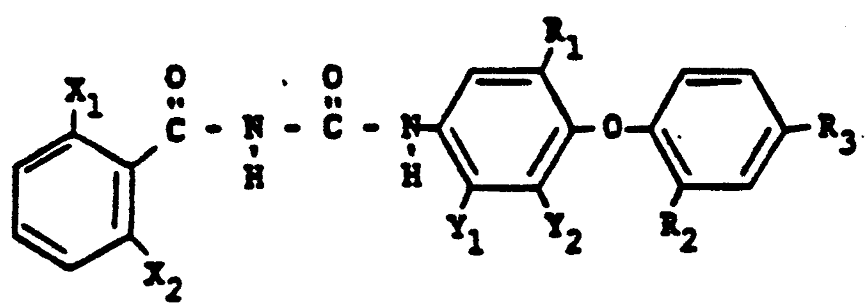

wherein $X_1$, $X_2$, $R_1$, $R_2$, $R_3$, $Y_1$ and $Y_2$ are as indicated above; and

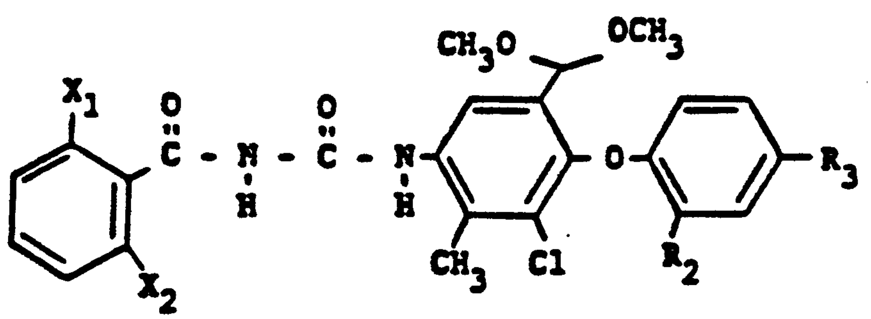

wherein $X_1$, $X_2$, $R_2$ and $R_3$ are as indicated above.

The following benzoyl urea compounds listed in Tables A through I are illustrative of those encompassed by the above Formula (1) and which can be prepared by the practice of this invention:

## Table A
## Representative 1-(4-Phenoxyphenyl)-3-Benzoyl Urea Compounds

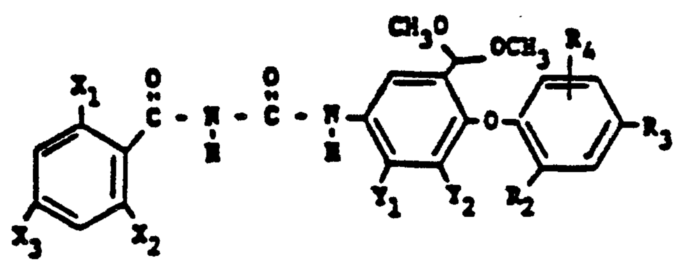

| $X_1$ | $X_2$ | $X_3$ | $Y_1$ | $Y_2$ | $R_2$ | $R_3$ | $R_4$ |
|---|---|---|---|---|---|---|---|
| F | F | H | $CH_3$ | Cl | Cl | Cl | H |
| Cl | H | H | $CH_3$ | Cl | Cl | Cl | H |
| F | H | H | $CH_3$ | Cl | Cl | Cl | H |
| F | F | F | $CH_3$ | Cl | Cl | Cl | H |
| F | Cl | H | $CH_3$ | Cl | Cl | Cl | H |
| F | F | H | $CH_3$ | Cl | Br | Cl | H |
| Cl | H | H | $CH_3$ | Cl | Br | Cl | H |
| F | H | H | $CH_3$ | Cl | Br | Cl | H |
| F | F | F | $CH_3$ | Cl | Br | Cl | H |
| F | H | F | $CH_3$ | Cl | Br | Cl | H |
| F | Cl | H | $CH_3$ | Cl | Br | Cl | H |
| F | F | H | $CH_3$ | Cl | $CH_3$ | $CH_3$ | H |
| Cl | H | H | $CH_3$ | Cl | $CH_3$ | $CH_3$ | H |
| F | H | H | $CH_3$ | Cl | $CH_3$ | $CH_3$ | H |
| F | F | F | $CH_3$ | Cl | $CH_3$ | $CH_3$ | H |
| F | Cl | H | $CH_3$ | Cl | $CH_3$ | $CH_3$ | H |
| F | F | H | $CH_3$ | Cl | Br | Br | H |

**Table A (Continued)**
**Representative 1-(4-Phenoxyphenyl)-3-**
**Benzoyl Urea Compounds**

| $X_1$ | $X_2$ | $X_3$ | $Y_1$ | $Y_2$ | $R_2$ | $R_3$ | $R_4$ |
|---|---|---|---|---|---|---|---|
| Cl | H | H | $CH_3$ | Cl | Br | Br | H |
| F | H | H | $CH_3$ | Cl | Br | Br | H |
| F | F | F | $CH_3$ | Cl | Br | Br | H |
| F | Cl | H | $CH_3$ | Cl | Br | Br | H |
| F | F | H | $CH_3$ | Cl | H | Cl | H |
| Cl | H | H | $CH_3$ | Cl | H | Cl | H |
| F | H | H | $CH_3$ | Cl | H | Cl | H |
| F | F | F | $CH_3$ | Cl | H | Cl | H |
| F | Cl | H | $CH_3$ | Cl | H | Cl | H |
| F | F | H | $CH_3$ | Cl | H | Cl | 3-Cl |
| Cl | H | H | $CH_3$ | Cl | H | Cl | 3-Cl |
| F | H | H | $CH_3$ | Cl | H | Cl | 3-Cl |
| F | F | F | $CH_3$ | Cl | H | Cl | 3-Cl |
| F | Cl | H | $CH_3$ | Cl | H | Cl | 3-Cl |
| F | F | H | $CH_3$ | Cl | $CH_3$ | Cl | 5-$CH_3$ |
| Cl | H | H | $CH_3$ | Cl | $CH_3$ | Cl | 5-$CH_3$ |
| F | H | H | $CH_3$ | Cl | $CH_3$ | Cl | 5-$CH_3$ |
| F | F | F | $CH_3$ | Cl | $CH_3$ | Cl | 5-$CH_3$ |
| F | Cl | H | $CH_3$ | Cl | $CH_3$ | Cl | 5-$CH_3$ |
| F | F | H | $CH_3$ | Cl | $CH_3$ | Br | 5-$CH_3$ |

## Table A (Continued)
## Representative 1-(4-Phenoxyphenyl)-3-Benzoyl Urea Compounds

| $X_1$ | $X_2$ | $X_3$ | $Y_1$ | $Y_2$ | $R_2$ | $R_3$ | $R_4$ |
|---|---|---|---|---|---|---|---|
| Cl | H | H | $CH_3$ | Cl | $CH_3$ | Br | 5-$CH_3$ |
| F | H | H | $CH_3$ | Cl | $CH_3$ | Br | 5-$CH_3$ |
| F | F | F | $CH_3$ | Cl | $CH_3$ | Br | 5-$CH_3$ |
| F | Cl | H | $CH_3$ | Cl | $CH_3$ | Br | 5-$CH_3$ |
| F | F | H | $CH_3$ | Cl | H | F | H |
| F | F | H | $CH_3$ | Cl | $CF_3$ | Cl | H |
| F | H | H | $CH_3$ | Cl | H | $CF_3$ | H |
| F | F | F | $CH_3$ | Cl | H | $CF_3$ | H |
| F | Cl | H | $CH_3$ | Cl | H | $CF_3$ | H |
| F | F | H | $CH_3$ | Cl | H | H | 3-$CF_3$ |
| F | F | F | $CH_3$ | Cl | H | H | 3-$CF_3$ |
| F | F | H | $CH_3$ | Cl | $CH_3$ | Br | H |
| Cl | H | H | $CH_3$ | Cl | $CH_3$ | Br | H |
| F | H | H | $CH_3$ | Cl | $CH_3$ | Br | H |
| F | F | F | $CH_3$ | Cl | $CH_3$ | Br | H |
| F | Cl | H | $CH_3$ | Cl | $CH_3$ | Br | H |
| F | F | H | H | Cl | Cl | Cl | H |
| Cl | H | H | H | Cl | Cl | Cl | H |
| F | F | F | H | Cl | Cl | Cl | H |
| F | F | H | H | Cl | Br | Cl | H |
| Cl | H | H | H | Cl | Br | Cl | H |

**Table A (Continued)**
**Representative 1-(4-Phenoxyphenyl)-3-**
**Benzoyl Urea Compounds**

| $X_1$ | $X_2$ | $X_3$ | $Y_1$ | $Y_2$ | $R_2$ | $R_3$ | $R_4$ |
|---|---|---|---|---|---|---|---|
| F | H | H | H | Cl | Br | Cl | H |
| F | F | F | H | Cl | Br | Cl | H |
| F | Cl | H | H | Cl | Br | Cl | H |
| F | F | H | H | Cl | H | Cl | H |
| Cl | H | H | H | Cl | H | Cl | H |
| F | F | F | H | Cl | H | Cl | H |
| F | F | H | $CH_3$ | Br | Cl | Cl | H |
| Cl | H | H | $CH_3$ | Br | Cl | Cl | H |
| F | H | H | $CH_3$ | Br | Cl | Cl | H |
| F | F | F | $CH_3$ | Br | Cl | Cl | H |
| F | Cl | H | $CH_3$ | Br | Cl | Cl | H |
| F | F | H | $CH_3$ | Br | Br | Cl | H |
| Cl | H | H | $CH_3$ | Br | Br | Cl | H |
| F | H | H | $CH_3$ | Br | Br | Cl | H |
| F | F | F | $CH_3$ | Br | Br | Cl | H |
| F | H | F | $CH_3$ | Br | Br | Cl | H |
| F | Cl | H | $CH_3$ | Br | Br | Cl | H |
| F | F | H | $CH_3$ | Br | H | Cl | H |
| Cl | H | H | $CH_3$ | Br | H | Cl | H |
| F | F | F | $CH_3$ | Br | H | Cl | H |

## Table A (Continued)
## Representative 1-(4-Phenoxyphenyl)-3-Benzoyl Urea Compounds

| $X_1$ | $X_2$ | $X_3$ | $Y_1$ | $Y_2$ | $R_2$ | $R_3$ | $R_4$ |
|---|---|---|---|---|---|---|---|
| F | F | H | $CH_3$ | H | Cl | Cl | H |
| Cl | H | H | $CH_3$ | H | Cl | Cl | H |
| F | F | F | $CH_3$ | H | Cl | Cl | H |
| F | Cl | H | $CH_3$ | H | Cl | Cl | H |
| F | F | H | $CH_3$ | H | Br | Cl | H |
| Cl | H | H | $CH_3$ | H | Br | Cl | H |
| F | H | H | $CH_3$ | H | Br | Cl | H |
| F | F | F | $CH_3$ | H | Br | Cl | H |
| F | H | F | $CH_3$ | H | Br | Cl | H |
| F | Cl | H | $CH_3$ | H | Br | Cl | H |
| F | F | H | Cl | Cl | Cl | Cl | H |
| Cl | H | H | Cl | Cl | Cl | Cl | H |
| F | F | F | Cl | Cl | Cl | Cl | H |
| F | F | H | Cl | Cl | Br | Cl | H |
| Cl | H | H | Cl | Cl | Br | Cl | H |
| F | H | H | Cl | Cl | Br | Cl | H |
| F | Cl | H | Cl | Cl | Br | Cl | H |
| F | F | H | Cl | Cl | H | Cl | H |
| Cl | H | H | Cl | Cl | H | Cl | H |
| F | F | H | H | $CH_3$ | Cl | Cl | H |

Table A (Continued)

Representative 1-(4-Phenoxyphenyl)-3-Benzoyl Urea Compounds

| $X_1$ | $X_2$ | $X_3$ | $Y_1$ | $Y_2$ | $R_2$ | $R_3$ | $R_4$ |
|---|---|---|---|---|---|---|---|
| Cl | H | H | H | $CH_3$ | Cl | Cl | H |
| F | F | F | H | $CH_3$ | Cl | Cl | H |
| F | F | H | H | $CH_3$ | Br | Cl | H |
| Cl | H | H | H | $CH_3$ | Br | Cl | H |
| F | H | H | H | $CH_3$ | Br | Cl | H |
| F | F | F | H | $CH_3$ | Br | Cl | H |
| F | F | H | H | $CH_3$ | H | Cl | H |
| Cl | H | H | H | $CH_3$ | H | Cl | H |
| F | F | F | H | $CH_3$ | H | Cl | H |
| F | Cl | H | H | $CH_3$ | H | Cl | H |
| F | F | H | Cl | $CH_3$ | Cl | Cl | H |
| Cl | H | H | Cl | $CH_3$ | Cl | Cl | H |
| F | F | F | Cl | $CH_3$ | Cl | Cl | H |
| F | F | H | Cl | $CH_3$ | Br | Cl | H |
| Cl | H | H | Cl | $CH_3$ | Br | Cl | H |
| F | H | H | Cl | $CH_3$ | Br | Cl | H |
| F | F | F | Cl | $CH_3$ | Br | Cl | H |
| F | H | F | Cl | $CH_3$ | Br | Cl | H |
| F | Cl | H | Cl | $CH_3$ | Br | Cl | H |

## Table B

## Representative 1-(4-Phenoxyphenyl)-3-Benzoyl Urea Compounds

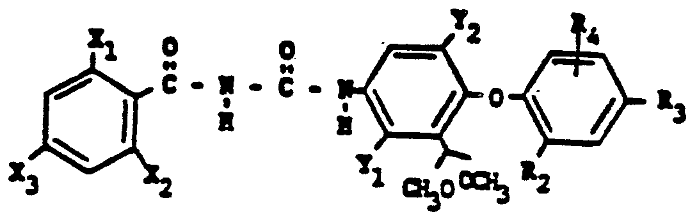

| $X_1$ | $X_2$ | $X_3$ | $Y_1$ | $Y_2$ | $R_2$ | $R_3$ | $R_4$ |
|---|---|---|---|---|---|---|---|
| F | F | H | $CH_3$ | H | Cl | Cl | H |
| F | F | H | $CH_3$ | H | $CH_3$ | $CH_3$ | H |
| F | F | F | $CH_3$ | H | $CH_3$ | $CH_3$ | H |
| F | F | H | $CH_3$ | H | Br | Cl | H |
| F | F | H | H | $CH_3$ | Br | Cl | H |
| F | F | H | Cl | Cl | Cl | Cl | H |
| F | F | H | Cl | Cl | H | $CF_3$ | H |
| F | F | F | Cl | Cl | H | $CF_3$ | H |
| F | F | H | Cl | Cl | Br | Cl | H |
| Cl | H | H | Cl | Cl | Br | Cl | H |
| F | H | H | Cl | Cl | Br | Cl | H |
| F | F | H | Cl | Cl | Cl | Cl | 5-$CH_3$ |
| F | F | H | Cl | Cl | F | F | H |
| F | F | H | Cl | Cl | Cl | H | 5-Cl |
| F | F | H | Cl | Cl | H | H | 3-$CF_3$ |

## Table C
## Representative 1-(4-Naphthoxyphenyl)-3-Benzoyl Urea Compounds

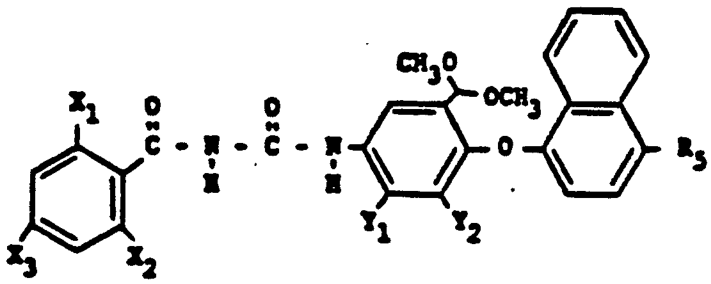

| $X_1$ | $X_2$ | $X_3$ | $Y_1$ | $Y_2$ | $R_5$ |
|---|---|---|---|---|---|
| F | F | H | $CH_3$ | Cl | Cl |
| Cl | H | H | $CH_3$ | Cl | Cl |
| F | F | H | $CH_3$ | Cl | H |
| F | F | F | $CH_3$ | Cl | Cl |
| F | Cl | H | $CH_3$ | Cl | Cl |
| F | F | H | $CH_3$ | Cl | Br |
| Cl | H | H | $CH_3$ | Cl | Br |
| F | F | F | $CH_3$ | Cl | Br |
| F | Cl | H | $CH_3$ | Cl | Br |
| F | F | H | $CH_3$ | Cl | $CH_3$ |
| F | F | F | $CH_3$ | Cl | $CH_3$ |
| F | F | H | H | Cl | Cl |
| Cl | H | H | H | Cl | Cl |

Table C (Continued)
Representative 1-(4-Naphthoxyphenyl)-3-
Benzoyl Urea Compounds

| $X_1$ | $X_2$ | $X_3$ | $Y_1$ | $Y_2$ | $R_5$ |
|---|---|---|---|---|---|
| F | F | F | H | Cl | Cl |
| F | F | H | $CH_3$ | H | Cl |
| Cl | H | H | $CH_3$ | H | Cl |
| F | F | F | $CH_3$ | H | Cl |
| F | Cl | H | $CH_3$ | H | Cl |
| F | F | H | $CH_3$ | Br | Cl |
| Cl | H | H | $CH_3$ | Br | Cl |
| F | F | F | $CH_3$ | Br | Cl |
| F | F | H | Cl | Cl | Cl |
| Cl | H | H | Cl | Cl | Cl |
| F | F | F | Cl | Cl | Cl |
| F | F | H | H | $CH_3$ | Cl |
| Cl | H | H | H | $CH_3$ | Cl |
| F | F | F | H | $CH_3$ | Cl |

## Table D

## Representative 1-(4-Tetrahydronaphthoxyphenyl)-3-Benzoyl Urea Compounds

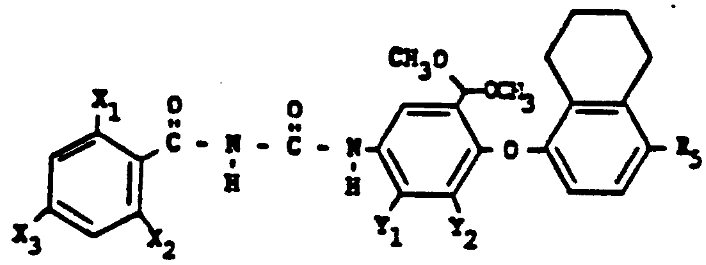

| $X_1$ | $X_2$ | $X_3$ | $Y_1$ | $Y_2$ | $R_5$ |
|---|---|---|---|---|---|
| F | F | H | $CH_3$ | Cl | Cl |
| Cl | H | H | $CH_3$ | Cl | Cl |
| F | F | F | $CH_3$ | Cl | H |
| F | F | F | $CH_3$ | Cl | Cl |
| F | Cl | H | $CH_3$ | Cl | Cl |
| F | F | H | $CH_3$ | Cl | Br |
| Cl | H | H | $CH_3$ | Cl | Br |
| F | F | F | $CH_3$ | Cl | Br |
| F | Cl | H | $CH_3$ | Cl | Br |
| F | F | H | $CH_3$ | Cl | $CH_3$ |
| F | F | F | $CH_3$ | Cl | $CH_3$ |
| F | F | H | H | Cl | Cl |
| Cl | H | H | H | Cl | Cl |
| F | F | F | H | Cl | Cl |
| F | F | H | $CH_3$ | H | Cl |
| Cl | H | H | $CH_3$ | H | Cl |
| F | F | F | $CH_3$ | H | Cl |
| F | Cl | H | $CH_3$ | H | Cl |
| F | F | H | Cl | $CH_3$ | Cl |

Table E

Representative 1-(4-Naphthoxyphenyl)-3-Benzoyl Urea Compounds

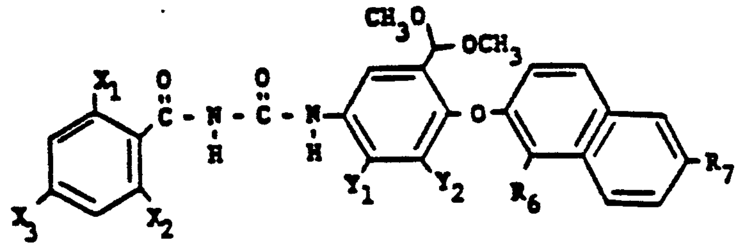

| $X_1$ | $X_2$ | $X_3$ | $Y_1$ | $Y_2$ | $R_6$ | $R_7$ |
|---|---|---|---|---|---|---|
| F | F | H | $CH_3$ | Cl | Br | Br |
| Cl | H | H | $CH_3$ | Cl | Br | Br |
| F | F | F | $CH_3$ | Cl | Br | Br |
| F | Cl | H | $CH_3$ | Cl | Br | Br |
| F | F | H | H | Cl | Br | Br |
| Cl | H | H | H | Cl | Br | Br |
| F | F | F | H | Cl | Br | Br |
| F | F | H | $CH_3$ | H | Br | Br |
| Cl | H | H | $CH_3$ | H | Br | Br |
| F | F | F | $CH_3$ | H | Br | Br |
| F | F | H | $CH_3$ | Br | Br | Br |
| Cl | H | H | $CH_3$ | Br | Br | Br |
| F | F | F | $CH_3$ | Br | Br | Br |
| F | F | H | Cl | Cl | Br | Br |
| F | F | F | Cl | Cl | Br | Br |
| F | F | H | H | $CH_3$ | Br | Br |
| F | F | H | $CH_3$ | Cl | H | H |
| Cl | H | H | $CH_3$ | Cl | H | H |
| F | F | F | $CH_3$ | Cl | H | H |

## Table F
## Representative 1-(4-Phenoxyphenyl)-3-Benzoyl Urea Compounds

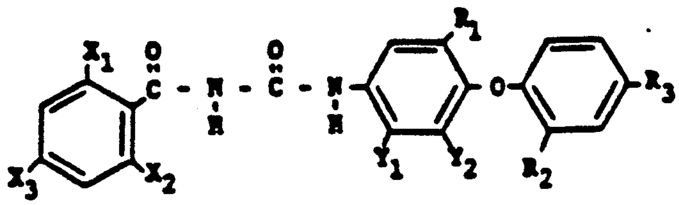

| $X_1$ | $X_2$ | $X_3$ | $Y_1$ | $Y_2$ | $R_1$ | $R_2$ | $R_3$ |
|---|---|---|---|---|---|---|---|
| F | F | H | $CH_3$ | Cl | $CO_2CH_3$ | Cl | Cl |
| Cl | H | H | $CH_3$ | Cl | $CO_2CH_3$ | Cl | Cl |
| F | F | H | Cl | $CH_3$ | $CO_2CH_3$ | Cl | H |
| F | F | H | $CH_3$ | Cl | $CO_2CH_3$ | Br | Cl |
| F | H | H | $CH_3$ | Cl | $CO_2CH_3$ | Br | Cl |
| F | F | F | $CH_3$ | Cl | $CO_2CH_3$ | Br | Cl |
| F | F | H | $CH_3$ | Cl | $CO_2CH_3$ | $CH_3$ | $CH_3$ |
| Cl | H | H | $CH_3$ | Cl | $CO_2CH_3$ | $CH_3$ | $CH_3$ |
| F | F | F | $CH_3$ | Cl | $CO_2CH_3$ | $CH_3$ | $CH_3$ |
| F | F | H | H | Cl | $CO_2CH_3$ | Cl | Cl |
| F | F | H | H | Cl | $CO_2CH_3$ | H | Cl |
| F | F | H | H | $CH_3$ | $CO_2CH_3$ | H | Cl |
| F | F | H | $CH_3$ | Cl | $CO_2CH_2CF_3$ | Cl | Cl |
| Cl | H | H | $CH_3$ | Cl | $CO_2CH_2CF_3$ | Cl | Cl |
| F | F | F | $CH_3$ | Cl | $CO_2CH_2CF_3$ | Cl | Cl |
| F | F | H | $CH_3$ | Cl | $CO_2CH_2CF_3$ | Br | Cl |

Table F (Continued)
Representative 1-(4-Phenoxyphenyl)-3-
Benzoyl Urea Compounds

| $X_1$ | $X_2$ | $X_3$ | $Y_1$ | $Y_2$ | $R_1$ | $R_2$ | $R_3$ |
|---|---|---|---|---|---|---|---|
| F | H | H | $CH_3$ | Cl | $CO_2CH_2CF_3$ | Br | Cl |
| F | F | F | $CH_3$ | Cl | $CO_2CH_2CF_3$ | Br | Cl |
| F | F | H | $CH_3$ | Cl | $CH(OCH_2)_2$ | Cl | Cl |
| Cl | H | H | $CH_3$ | Cl | $CH(OCH_2)_2$ | Cl | Cl |
| F | Cl | H | $CH_3$ | Cl | $CH(OCH_2)_2$ | Cl | Cl |
| F | F | H | $CH_3$ | Cl | $CH(OCH_2)_2$ | Br | Cl |
| F | H | H | $CH_3$ | Cl | $CH(OCH_2)_2$ | Br | Cl |
| F | Cl | H | $CH_3$ | Cl | $CH(OCH_2)_2$ | Br | Cl |
| F | F | F | $CH_3$ | Cl | $CH(OCH_2)_2$ | Br | Cl |
| F | F | H | $CH_3$ | Cl | $CH[O(CH_2)_3O]$ | Cl | Cl |
| Cl | H | H | $CH_3$ | Cl | $CH[O(CH_2)_3O]$ | Cl | Cl |
| F | F | F | $CH_3$ | Cl | $CH[O(CH_2)_3O]$ | Cl | Cl |
| F | F | H | $CH_3$ | Cl | $CH[O(CH_2)_3O]$ | Br | Cl |
| Cl | H | H | $CH_3$ | Cl | $CH[O(CH_2)_3O]$ | Br | Cl |
| F | H | H | $CH_3$ | Cl | $CH[O(CH_2)_3O]$ | Br | Cl |
| F | F | F | $CH_3$ | Cl | $CH[O(CH_2)_3O]$ | Br | Cl |
| F | F | H | $CH_3$ | Cl | $CH[O(CH_2)_3O]$ | $CH_3$ | $CH_3$ |
| F | F | H | $CH_3$ | Cl | $CH[O(CH_2)_3O]$ | H | Cl |
| F | F | H | $CH_3$ | Cl | $CO_2H$ | Cl | Cl |
| F | F | H | $CH_3$ | Cl | $CO_2H$ | Br | Cl |

Table F (Continued)
Representative 1-(4-Phenoxyphenyl)-3-Benzoyl Urea Compounds

| $X_1$ | $X_2$ | $X_3$ | $Y_1$ | $Y_2$ | $R_1$ | $R_2$ | $R_3$ |
|---|---|---|---|---|---|---|---|
| F | F | H | $CH_3$ | Cl | $CO_2H$ | $CH_3$ | $CH_3$ |
| F | F | H | $CH_3$ | Cl | $CH_2OH$ | Cl | Cl |
| F | F | F | $CH_3$ | Cl | $CH_2OH$ | Cl | Cl |
| F | F | H | $CH_3$ | Cl | $CH_2OH$ | Br | Cl |
| F | H | H | $CH_3$ | Cl | $CH_2OH$ | Br | Cl |
| F | H | F | $CH_3$ | Cl | $CH_2OH$ | Br | Cl |
| F | F | F | $CH_3$ | Cl | $CH_2OH$ | Br | Cl |
| F | F | H | $CH_3$ | Cl | $CH_2OCCl{=}CF_2$ | Br | Cl |
| Cl | H | H | $CH_3$ | Cl | $CH_2OCCl{=}CF_2$ | Br | Cl |
| F | F | H | $CH_3$ | Cl | $CH(OCH_2CH_3)_2$ | Br | Cl |
| F | F | F | $CH_3$ | Cl | $CH(OCH_2CH_3)_2$ | Br | Cl |
| F | Cl | H | $CH_3$ | Cl | $CH_2OCH_3$ | Br | Cl |
| F | F | H | $CH_3$ | Cl | $CH_2OCH_3$ | Br | Cl |
| Cl | H | H | $CH_3$ | Cl | $CH_2OCH_3$ | Br | Cl |
| F | H | H | $CH_3$ | Cl | $CH_2OCH_3$ | Br | Cl |
| F | F | F | $CH_3$ | Cl | $CH_2OCH_3$ | Br | Cl |
| F | H | F | $CH_3$ | Cl | $CH_2OCH_3$ | Br | Cl |
| F | F | H | $CH_3$ | Cl | $CH_2OCH_3$ | $CH_3$ | $CH_3$ |
| F | F | F | $CH_3$ | Cl | $CH_2OCH_3$ | $CH_3$ | $CH_3$ |
| F | F | H | $CH_3$ | Cl | $CH_2OCH_3$ | H | Cl |

Table F (Continued)
Representative 1-(4-Phenoxyphenyl)-3-Benzoyl Urea Compounds

| $X_1$ | $X_2$ | $X_3$ | $Y_1$ | $Y_2$ | $R_1$ | $R_2$ | $R_3$ |
|---|---|---|---|---|---|---|---|
| F | F | H | $CH_3$ | Cl | $CH_2OCH_3$ | F | F |
| F | F | H | H | Cl | $CH_2OCH_3$ | Cl | Cl |
| F | F | H | H | Cl | $CH_2OCH_3$ | Br | Cl |
| F | F | H | $CH_3$ | Cl | $CH_2OCH_2CF_3$ | Cl | Cl |
| Cl | H | H | $CH_3$ | Cl | $CH_2OCH_2CF_3$ | Cl | Cl |
| F | F | F | $CH_3$ | Cl | $CH_2OCH_2CF_3$ | Cl | Cl |
| F | F | H | $CH_3$ | Cl | $CH_2OCH_2CF_3$ | Br | Cl |
| Cl | H | H | $CH_3$ | Cl | $CH_2OCH_2CF_3$ | Br | Cl |
| F | H | H | $CH_3$ | Cl | $CH_2OCH_2CF_3$ | Br | Cl |
| F | F | F | $CH_3$ | Cl | $CH_2OCH_2CF_3$ | Br | Cl |
| F | Cl | H | $CH_3$ | Cl | $CH_2OCH_2CF_3$ | Br | Cl |
| F | F | H | $CH_3$ | Cl | $CH_2OCOCH_3$ | Cl | Cl |
| F | F | H | $CH_3$ | Cl | $CH_2OCOCH_3$ | Br | Cl |
| F | F | H | $CH_3$ | Br | CHO | Cl | Cl |
| F | F | H | $CH_3$ | Cl | CHO | Cl | Cl |
| F | F | H | $CH_3$ | Cl | CHO | Br | Cl |
| F | F | H | $CH_3$ | Cl | $CH{=}CHCOOCH_3$ | Cl | Cl |
| F | F | H | $CH_3$ | Cl | $CH{=}CHCOOCH_3$ | $CH_3$ | $CH_3$ |
| F | F | F | $CH_3$ | Cl | $CH{=}CHCOOCH_3$ | $CH_3$ | $CH_3$ |
| F | F | H | $CH_3$ | Cl | $CH{=}CHCOOCH_3$ | Br | Cl |

**Table F (Continued)**
**Representative 1-(4-Phenoxyphenyl)-3-Benzoyl Urea Compounds**

| $X_1$ | $X_2$ | $X_3$ | $Y_1$ | $Y_2$ | $R_1$ | $R_2$ | $R_3$ |
|---|---|---|---|---|---|---|---|
| F | H | H | $CH_3$ | Cl | $CH{=}CHCOOCH_3$ | Br | Cl |
| F | F | F | $CH_3$ | Cl | $CH{=}CHCOOCH_3$ | Br | Cl |
| F | F | H | $CH_3$ | Cl | $CH{=}C(Cl)_2$ | Cl | Cl |
| Cl | H | H | $CH_3$ | Cl | $CH{=}C(Cl)_2$ | Cl | Cl |
| F | F | F | $CH_3$ | Cl | $CH{=}C(Cl)_2$ | Cl | Cl |
| F | Cl | H | $CH_3$ | Cl | $CH{=}C(Cl)_2$ | Cl | Cl |
| F | F | H | $CH_3$ | Cl | $CH{=}C(Br)_2$ | Cl | Cl |
| F | F | H | $CH_3$ | Cl | $CH{=}C(Br)_2$ | Br | Cl |
| F | F | H | $CH_3$ | Cl | $CH{=}CHNO_2$ | Cl | Cl |
| F | F | H | $CH_3$ | Cl | $CH{=}CHNO_2$ | Br | Cl |
| F | F | H | $CH_3$ | Cl | $CH{=}CHCN$ | Cl | Cl |
| F | F | H | $CH_3$ | Cl | $CH{=}CHCN$ | Br | Cl |
| F | F | H | $CH_3$ | Cl | $CH{=}C(COOCH_3)_2$ | Cl | Cl |
| F | F | H | $CH_3$ | Cl | $CH{=}C(COOCH_3)_2$ | Br | Cl |
| F | F | H | $CH_3$ | H | $CH_2OCH_3$ | H | $NO_2$ |
| F | F | H | $CH_3$ | H | $CH_2OCH_3$ | Cl | $NO_2$ |
| F | F | H | $CH_3$ | H | $CH_2OCH_3$ | H | CN |
| F | F | H | $CH_3$ | H | $CH_2OCH_3$ | Cl | $CF_3$ |
| Cl | H | H | $CH_3$ | H | $CH_2OCH_3$ | Cl | $CF_3$ |

**Table F (Continued)**
**Representative 1-(4-Phenoxyphenyl)-3-**
**Benzoyl Urea Compounds**

| $X_1$ | $X_2$ | $X_3$ | $Y_1$ | $Y_2$ | $R_1$ | $R_2$ | $R_3$ |
|---|---|---|---|---|---|---|---|
| F | F | H | $CH_3$ | H | $CH_2OCH_3$ | $CF_3$ | Cl |
| F | F | F | $CH_3$ | H | $CH_2OCH_3$ | Cl | $CF_3$ |
| F | Cl | H | $CH_3$ | H | $CH_2OCH_3$ | Cl | $CF_3$ |
| Cl | H | H | $CH_3$ | Cl | $CH(OCH_2)_2$ | Br | Cl |
| F | H | H | $CH_3$ | Cl | $CH(OCH_2)_2$ | Cl | Cl |
| F | F | H | $CH_3$ | Cl | $CH_2OCH_3$ | Cl | Cl |
| Cl | H | H | $CH_3$ | Cl | $CO_2CH_3$ | Br | Cl |
| F | F | H | $CH_3$ | Cl | $CO_2CH_2CH_3$ | Br | Cl |
| F | H | H | $CH_3$ | Cl | $CO_2CH_2CH_3$ | Br | Cl |
| F | F | H | $CH_3$ | Cl | $CO_2CH_2CH(CH_3)_2$ | Br | Cl |
| F | H | H | $CH_3$ | Cl | $CO_2CH_2CH(CH_3)_2$ | Br | Cl |
| F | F | H | $CH_3$ | Cl | $CO_2CH_2CH{=}CH_2$ | Br | Cl |
| F | F | H | $CH_3$ | Cl | $CO_2CH_2C{\equiv}CH$ | Br | Cl |
| F | F | H | $CH_3$ | Cl | $CO_2CH_2CH{=}CCl_2$ | Br | Cl |
| F | F | H | $CH_3$ | Cl | $CH_2OCH_2C{\equiv}CH$ | Br | Cl |
| F | F | H | $CH_3$ | Cl | $CH_2OCH_2CH(CH_3)_2$ | Br | Cl |
| F | F | H | $CH_3$ | Cl | $CH_2OCH_2CH_2OC_2H_5$ | Br | Cl |
| F | F | H | $CH_3$ | Cl | $CH(OCH_2CH_2CH_2CH_3)_2$ | Br | Cl |
| F | F | H | $CH_3$ | Cl | $CH_2OCH_2CH_2Cl$ | Br | Cl |

## Table F (Continued)
## Representative 1-(4-Phenoxyphenyl)-3-Benzoyl Urea Compounds

| $X_1$ | $X_2$ | $X_3$ | $Y_1$ | $Y_2$ | $R_1$ | $R_2$ | $R_3$ |
|---|---|---|---|---|---|---|---|
| F | F | H | $CH_3$ | Cl | $CH_2O(CH_2)_4CH_3$ | Br | Cl |
| F | F | H | $CH_3$ | Cl | $CH[OCH(CH_2CH_3)C_3H_7]_2$ | Br | Cl |
| F | F | H | $CH_3$ | Cl | $C(O)O-n-C_6H_{13}$ | Br | Cl |
| F | F | H | $CH_3$ | Cl | $CH=CHCH_3$ | Cl | Cl |
| F | F | H | $CH_3$ | Cl | $CH=C(CH_3)_2$ | Cl | Cl |
| F | F | H | $CH_3$ | Cl | $CH=CH(CH_2)_3CH_3$ | Cl | Cl |
| F | F | H | $CH_3$ | Cl | $CH(SCH_2)_2$ | Cl | Cl |
| Cl | H | H | $CH_3$ | Cl | $CH(SCH_2)_2$ | Cl | Cl |
| F | F | H | $CH_3$ | Cl | $CH(SCH_2CH_2O)$ | Cl | Cl |
| Cl | H | H | $CH_3$ | Cl | $CH(SCH_2CH_2O)$ | Cl | Cl |
| F | F | H | $CH_3$ | Cl | $CH(SCH_2)_2$ | F | F |
| Cl | H | H | $CH_3$ | Cl | $CH(SCH_2)_2$ | F | F |
| F | F | H | $CH_3$ | Cl | $CH(SCH_3)_2$ | Cl | Cl |
| F | F | H | $CH_3$ | Cl | $CH(SCH_3)_2$ | Br | Cl |
| F | F | H | $CH_3$ | Cl | $CH(SCH_3)_2$ | F | F |
| F | F | H | $CH_3$ | Cl | $CH(SCH_2)_2$ | Br | Cl |
| F | F | H | $CH_3$ | Cl | $CH[S(CH_2)_3S]$ | Br | Cl |
| F | F | H | $CH_3$ | Cl | $CH(SCH_2CH_2O)$ | Br | Cl |

## Table G

## Representative 1-(4-Naphthoxyphenyl)-3-Benzoyl Urea Compounds

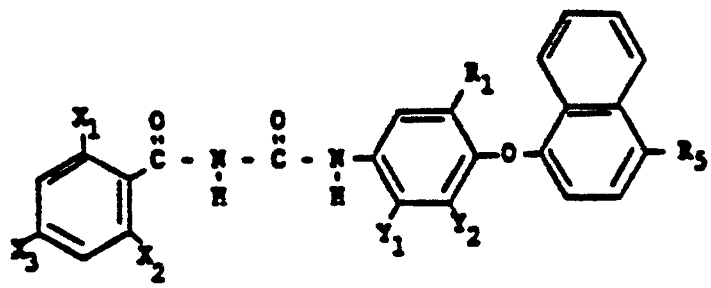

| $X_1$ | $X_2$ | $X_3$ | $Y_1$ | $Y_2$ | $R_1$ | $R_5$ |
|---|---|---|---|---|---|---|
| F | F | H | $CH_3$ | Cl | $COOCH_3$ | Cl |
| Cl | H | H | $CH_3$ | Cl | $COOCH_3$ | Cl |
| F | Cl | H | $CH_3$ | Cl | $COOCH_3$ | Cl |
| F | F | F | $CH_3$ | Cl | $COOCH_3$ | Cl |
| F | F | H | $CH_3$ | Cl | $CH_2OCH_3$ | Cl |
| Cl | H | H | $CH_3$ | Cl | $CH_2OCH_3$ | Cl |
| F | F | F | $CH_3$ | Cl | $CH_2OCH_3$ | Cl |
| F | F | H | H | $CH_3$ | $CH_2OCH_3$ | Cl |
| Cl | H | H | H | $CH_3$ | $CH_2OCH_3$ | Cl |
| F | F | H | $CH_3$ | H | $CH_2OCH_3$ | Cl |
| F | F | H | Cl | Cl | $CH_2OCH_3$ | Cl |
| Cl | H | H | Cl | Cl | $CH_2OCH_3$ | Cl |
| F | F | H | $CH_3$ | Cl | $CH_2OCH_3$ | $CH_3$ |
| F | F | H | $CH_3$ | Cl | $CH_2OCH_3$ | H |
| F | F | H | $CH_3$ | Cl | $CH(OCH_2CH_3)_2$ | Cl |
| F | F | H | $CH_3$ | Cl | $CH(OCH_2)_2$ | Cl |

**Table G (Continued)**
**Representative 1-(4-Naphthoxyphenyl)-3-Benzoyl Urea Compounds**

| $X_1$ | $X_2$ | $X_3$ | $Y_1$ | $Y_2$ | $R_1$ | $R_5$ |
|---|---|---|---|---|---|---|
| F | F | H | $CH_3$ | Cl | $CH(O-(CH_2)_3-O)$ | Cl |
| F | F | H | $CH_3$ | Cl | $CO_2H$ | Cl |
| F | F | H | $CH_3$ | Cl | $CH_2OH$ | Cl |
| F | F | H | $CH_3$ | Cl | $CH_2OCOCH_3$ | Cl |
| F | F | H | $CH_3$ | Cl | CHO | Cl |
| F | F | H | $CH_3$ | Cl | $CH_2OCCl=C(F)_2$ | Cl |
| F | F | H | $CH_3$ | Cl | $CH=C(Cl)_2$ | Cl |
| F | F | H | $CH_3$ | Cl | $CH=CHNO_2$ | Cl |
| F | F | H | $CH_3$ | Cl | CH=CHCN | Cl |
| F | F | H | $CH_3$ | Cl | $CH=CHCOOCH_3$ | Cl |
| F | F | H | $CH_3$ | Cl | $CH(SCH_2)_2$ | Cl |
| Cl | H | H | $CH_3$ | Cl | $CH(SCH_2)_2$ | Cl |
| F | F | H | $CH_3$ | Cl | $CH(SCH_2CH_2O)$ | Cl |
| F | F | H | $CH_3$ | Cl | $CH(SCH_3)_2$ | Cl |

## Table H

## Representative 1-(4-Tetrahydronaphthoxyphenyl)-3-Benzoyl Urea Compounds

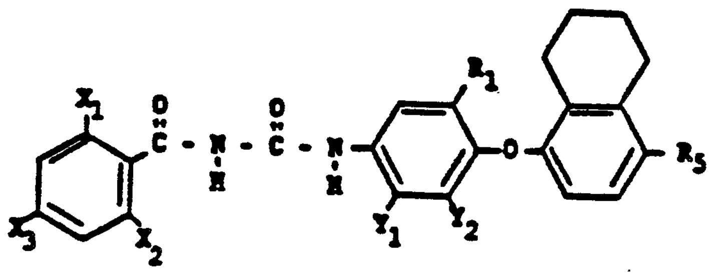

| $X_1$ | $X_2$ | $X_3$ | $Y_1$ | $Y_2$ | $R_1$ | $R_5$ |
|---|---|---|---|---|---|---|
| F | F | H | $CH_3$ | Cl | $CO_2CH_3$ | Cl |
| Cl | H | H | $CH_3$ | Cl | $CO_2CH_3$ | Cl |
| F | Cl | H | $CH_3$ | Cl | $CO_2CH_3$ | Cl |
| F | F | F | $CH_3$ | Cl | $CO_2CH_3$ | Cl |
| F | F | H | $CH_3$ | Cl | $CH_2OCH_3$ | Cl |
| Cl | H | H | $CH_3$ | Cl | $CH_2OCH_3$ | Cl |
| F | F | F | $CH_3$ | Cl | $CH_2OCH_3$ | Cl |
| F | F | H | H | Br | $CH_2OCH_3$ | Cl |
| F | F | H | $CH_3$ | Br | $CH_2OCH_3$ | Cl |
| F | F | F | $CH_3$ | Br | $CH_2OCH_3$ | Cl |
| F | F | H | $CH_3$ | $CH_3$ | $CH_2OCH_3$ | Cl |
| F | F | H | $CH_3$ | H | $CH_2OCH_3$ | Cl |
| Cl | H | H | $CH_3$ | H | $CH_2OCH_3$ | Cl |
| F | F | H | $CH_3$ | H | $CH_2OCH_3$ | $CH_3$ |
| F | F | H | Cl | Cl | $CH_2OCH_3$ | Cl |
| F | F | H | $CH_3$ | Cl | $CH_2(OCH_3)_2$ | Cl |
| F | F | H | $CH_3$ | Cl | $CH(SCH_2)_2$ | Cl |
| F | F | H | $CH_3$ | Cl | $CH(SCH_3)_2$ | Cl |
| F | F | H | $CH_3$ | Cl | $CH(SCH_2CH_2O)$ | Cl |

## Table H (Continued)
## Representative 1-(4-Tetrahydronaphthoxyphenyl)-3-Benzoyl Urea Compounds

| $X_1$ | $X_2$ | $X_3$ | $Y_1$ | $Y_2$ | $R_1$ | $R_5$ |
|---|---|---|---|---|---|---|
| F | F | H | $CH_3$ | Cl | $CH(OCH_2CH_3)_2$ | Cl |
| F | F | H | $CH_3$ | Cl | $CH(OCH_2)_2$ | Cl |
| F | F | H | $CH_3$ | Cl | $CH(O-(CH_2)_3-O)$ | Cl |
| F | F | H | $CH_3$ | Cl | $CO_2H$ | Cl |
| F | F | H | $CH_3$ | Cl | $CH_2OH$ | Cl |
| F | F | H | $CH_3$ | Cl | $CH_2OCOCH_3$ | Cl |
| F | F | H | $CH_3$ | Cl | CHO | Cl |
| F | F | H | $CH_3$ | Cl | $CH_2OCCl=C(F)_2$ | Cl |
| F | F | H | $CH_3$ | Cl | $CH=C(Cl)_2$ | Cl |
| F | F | H | $CH_3$ | Cl | $CH=CHNO_2$ | Cl |
| F | F | H | $CH_3$ | Cl | CH=CHCN | Cl |
| F | F | H | $CH_3$ | Cl | $CH=CHCO_2CH_3$ | Cl |

## Table I

## Representative 1-(4-Naphthoxyphenyl)-3-Benzoyl Urea Compounds

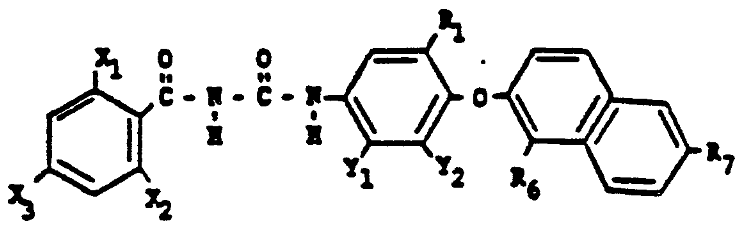

| $X_1$ | $X_2$ | $X_3$ | $Y_1$ | $Y_2$ | $R_1$ | $R_6$ | $R_7$ |
|---|---|---|---|---|---|---|---|
| F | F | H | $CH_3$ | Cl | $CO_2CH_3$ | Br | Br |
| Cl | H | H | $CH_3$ | Cl | $CO_2CH_3$ | Br | Br |
| F | F | H | H | $CH_3$ | $CO_2CH_3$ | Br | Br |
| F | F | H | $CH_3$ | Cl | $CH_2OCH_3$ | Br | Br |
| Cl | H | H | $CH_3$ | Cl | $CH_2OCH_3$ | Br | Br |
| F | F | F | $CH_3$ | Cl | $CH_2OCH_3$ | Br | Br |
| F | F | H | $CH_3$ | Br | $CH_2OCH_3$ | Br | Br |
| Cl | H | H | $CH_3$ | Br | $CH_2OCH_3$ | Br | Br |
| F | F | F | $CH_3$ | Br | $CH_2OCH_3$ | Br | Br |
| F | F | H | $CH_3$ | H | $CH_2OCH_3$ | Br | Br |
| F | F | H | H | Cl | $CH_2OCH_3$ | Br | Br |
| F | F | H | Cl | Cl | $CH_2OCH_3$ | Br | Br |
| Cl | H | H | Cl | Cl | $CH_2OCH_3$ | Br | Br |
| F | F | H | $CH_3$ | Cl | $CH_2OCH_3$ | H | H |
| F | F | H | $CH_3$ | Br | $CH_2OCH_3$ | H | H |
| F | F | H | $CH_3$ | Cl | $CH_2OCCl=C(F)_2$ | H | H |

## Table I (Continued)
## Representative 1-(4-Naphthoxyphenyl)-3-Benzoyl Urea Compounds

| $X_1$ | $X_2$ | $X_3$ | $Y_1$ | $Y_2$ | $R_1$ | $R_6$ | $R_7$ |
|---|---|---|---|---|---|---|---|
| F | F | H | $CH_3$ | Cl | $CH(OCH_2CH_3)_2$ | Br | Br |
| F | F | H | $CH_3$ | Cl | $CH(OCH_2)_2$ | Br | Br |
| F | F | H | $CH_3$ | Cl | $CH(O(-CH_2)_3-O)$ | Br | Br |
| F | F | H | $CH_3$ | Cl | $CO_2H$ | Br | Br |
| F | F | H | $CH_3$ | Cl | $CH_2OH$ | Br | Br |
| F | F | H | $CH_3$ | Cl | $CH_2OCH_2CF_3$ | Br | Br |
| F | F | H | $CH_3$ | Cl | $CH_2OCOCH_3$ | Br | Br |
| F | F | H | $CH_3$ | Cl | CHO | Br | Br |
| F | F | H | $CH_3$ | Cl | $CH=C(Cl)_2$ | Br | Br |
| F | F | H | $CH_3$ | Cl | $CH=CHNO_2$ | Br | Br |
| F | F | H | $CH_3$ | Cl | CH=CHCN | Br | Br |
| F | F | H | $CH_3$ | Cl | $CH=CHCOOCH_3$ | Br | Br |
| F | F | H | $CH_3$ | Cl | $CH(SCH_2)_2$ | Br | Br |
| F | F | H | $CH_3$ | Cl | $CH(SCH_2CH_2O)$ | Br | Br |
| F | F | H | $CH_3$ | Cl | $CH(SCH_3)_2$ | Br | Br |

The novel benzoyl urea compounds of this invention encompassed by Formula (1) can be conveniently prepared by one or more methods. For example, the compounds of this invention may be prepared by reacting a substituted aryloxyaniline 2 with a benzoyl isocyanate 3 according to Scheme I as follows:

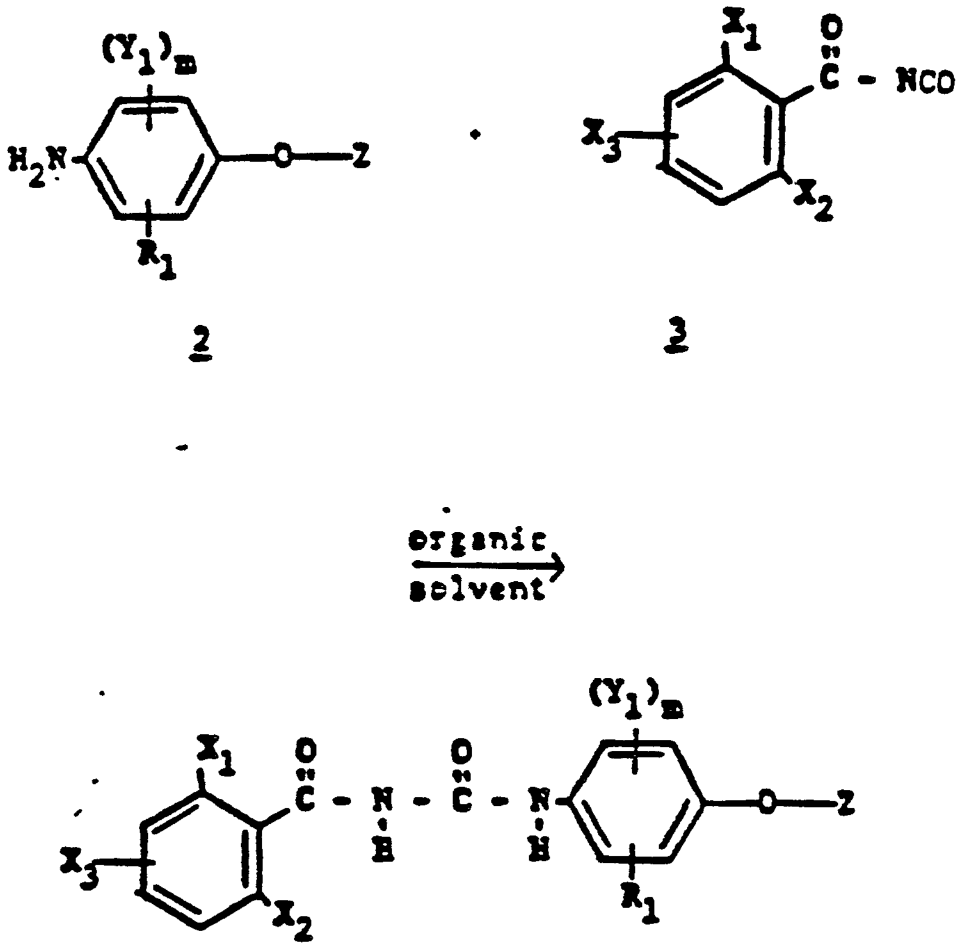


2

3

Scheme I

wherein $X_1$, $X_2$, $X_3$, $Y_1$, m, $R_1$ and Z have the meaning given in Formula (1).

Alternatively, the novel compounds may be prepared by the reaction of an aryloxyphenylisocyanate 4 with a benzamide 5 according to Scheme II as follows:

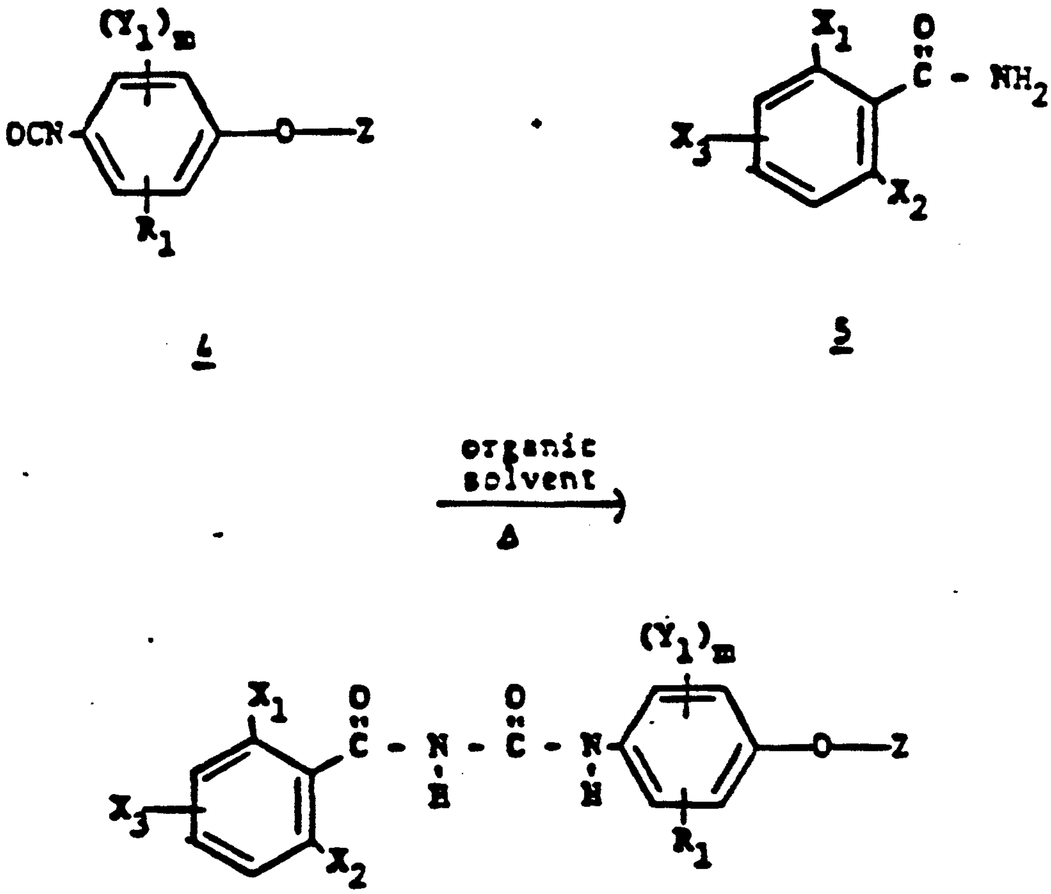


4

5

Scheme II

wherein $X_1$, $X_2$, $X_3$, $Y_1$, m, $R_1$ and Z have the meaning given in Formula (1) except $R_1$ is not a hydroxyalkyl group or carboxylic acid group.

The subject compounds may also be prepared by the reaction of a benzoyl chloride 6 with a substituted urea 7 according to Scheme III as follows:

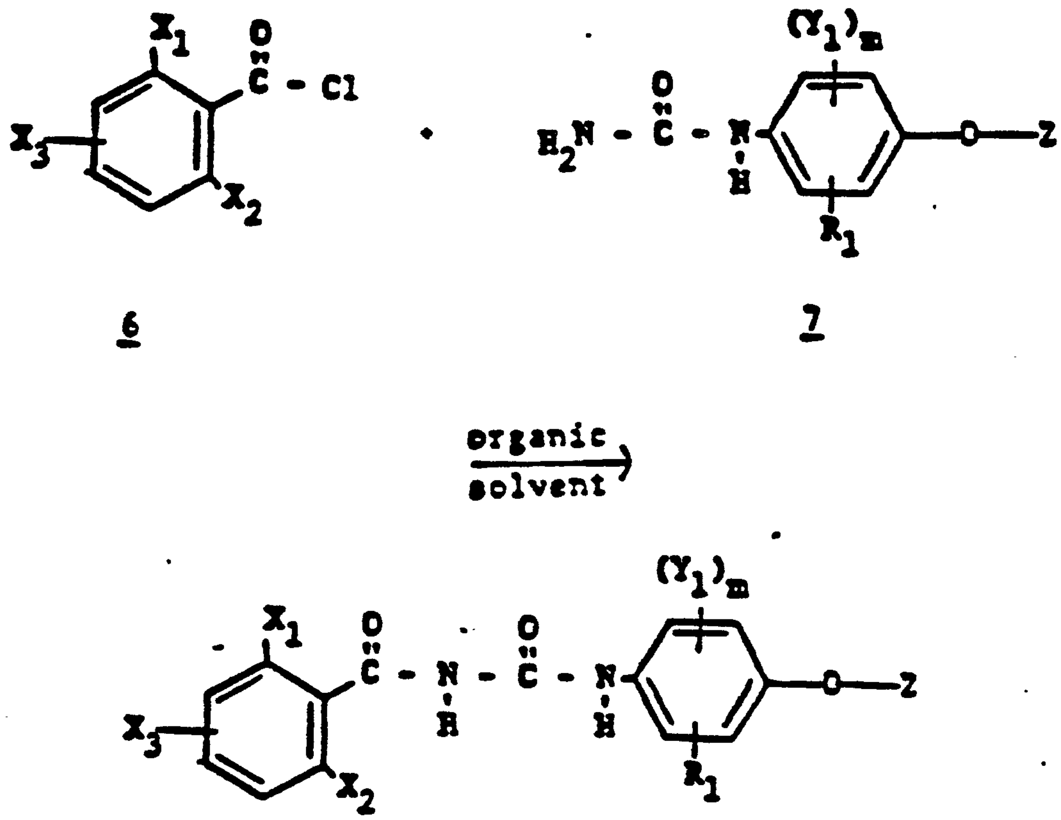


6

7

Scheme III

wherein $X_1$, $X_2$, $X_3$, $Y_1$, m, $R_1$ and Z have the meaning given in Formula (1) except $R_1$ is not an acyclic or cyclic acetal group, aldehyde group, carboxylic acid group or hydroxyalkyl group.

In general, the reactions illustrated in Schemes I, II and III can be carried out in organic solvents such as aromatic hydrocarbons, halogenated hydrocarbons, ethers and the like. Solvents like toluene, 1,2-dichloroethane, dichloromethane and p-dioxane are preferred. These reactions proceed at temperatures ranging from ambient temperature to about 150°C or the reflux temperature of the chosen solvent.

The intermediates shown in Schemes I, II and III can be prepared according to generally accepted procedures. Thus, the substituted benzoyl isocyanate 3 can be prepared from the corresponding benzamide 5 following the general procedure of Speziale et. al., J. Org. Chem. 27, 3742 (1962) as follows:

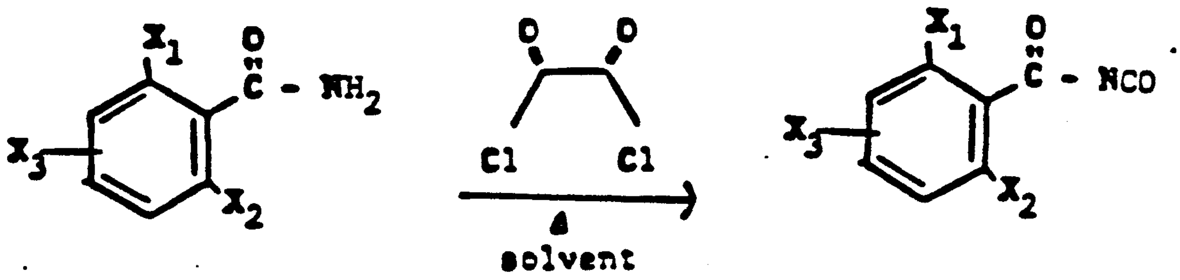


5 3

## Scheme IV

wherein $X_1$, $X_2$ and $X_3$ have the meaning given in Formula (1).

The substituted aryloxyanilines 2 may be prepared according to Scheme V involving the reaction of a substituted aromatic alcohol 9 with a chloronitrobenzene 8 as follows:

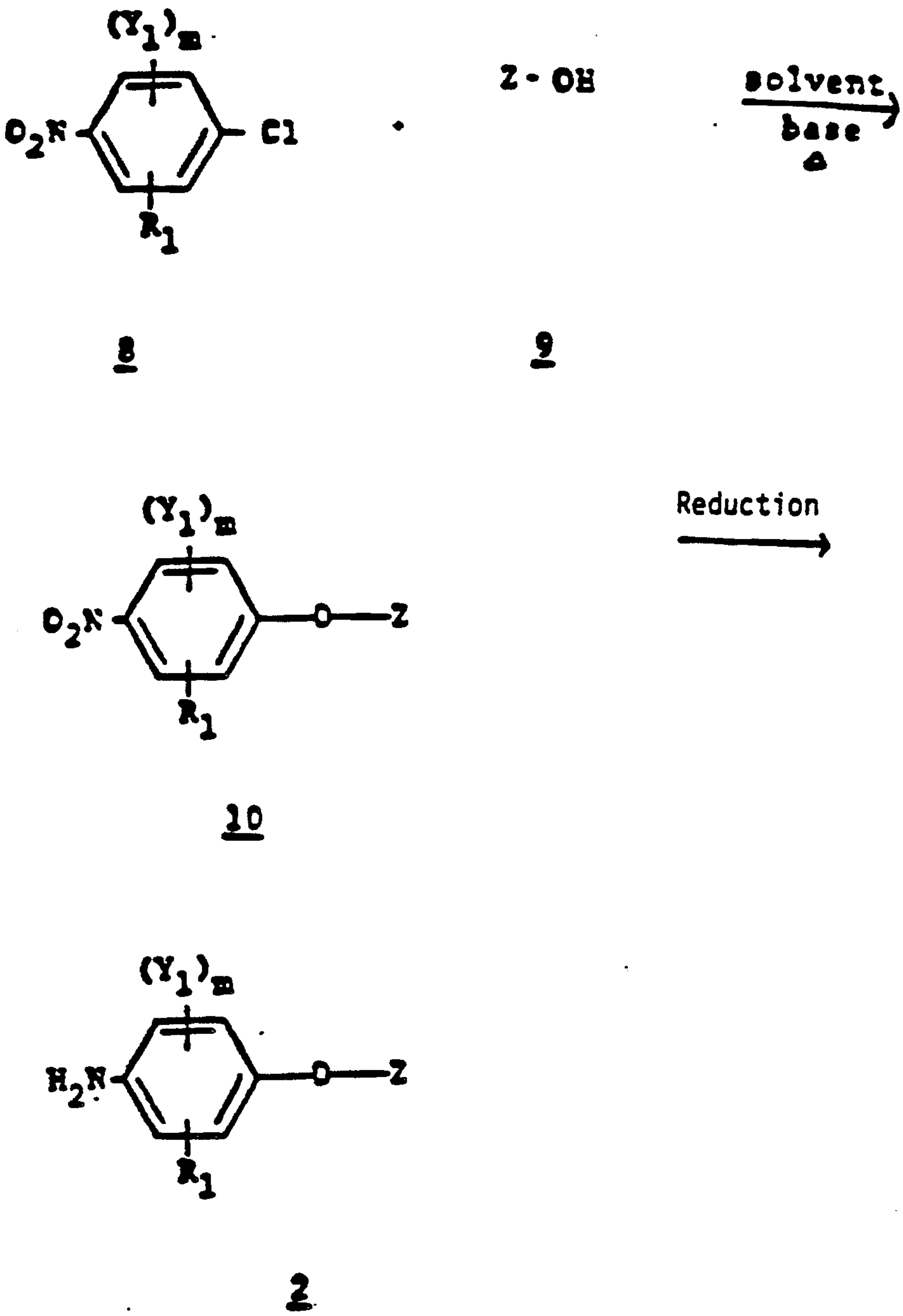


8 9

10

2

Scheme V

wherein $Y_1$, m, $R_1$ and Z have the meaning given in Formula (1) except $R_1$ is not an acyloxyalkyl group and provided that when $Y_1$ is ortho to the nitro group then it is alkyl. The reaction of a substituted aromatic alcohol 9 with a chloronitrobenzene 8 to give the aryloxynitrobenzene 10 proceeds in the presence of a base in an inert solvent at elevated temperature. Bases suitable for this reaction are potassium carbonate, sodium hydride, potassium hydroxide, and sodium hydroxide. Suitable solvents are toluene, dimethylformamide, and dimethylsulfoxide. The above transformation can be carried out in a diphasic reaction medium in the presence of a phase-transfer catalyst.

The reduction of aryloxynitrobenzene 10 to aryloxyaniline 2 can be achieved under a hydrogen atmosphere using a heterogeneous or homogeneous catalytic system. Suitable catalysts include palladium or platinum on a inert support, a Raney nickel catalyst or an organometallic catalyst such as dichlorotris-(triphenylphosphine) ruthenium (Tetrahedron Letters, 2163 (1975)). The generally preferred conditions for these reductions involve hydrogenation at a hydrogen pressure of 40-100 psi in an inert solvent at ambient

temperature. Solvents of choice include aromatic hydrocarbons such as toluene, alcohols such as ethanol or esters such as ethyl acetate. Alternatively, this reduction may be accomplished by a chemical reductant such as a transition metal or its salts in a mineral acid solution. In general tin or iron and their salts in hydrochloric acid are preferred. A co-solvent such as dioxane or alcohols may be added to improve the reactant solubility. Other suitable reducing systems are illustrated by the use of ammonium formate in methanol in the presence of a palladium catalyst at room temperature; Tetrahedron Letters 3415, (1984), the use of hydrazine and a metal catalyst; Can. J.Chem., 38, 2363 (1960), and reduction with stannous chloride in ethanol; Tetrahedron Letters 839, (1984).

Isocyanate 4 can be obtained by reacting the substituted aryloxyaniline 2 with phosgene or upon exposure of the substituted aryloxyaniline 2 to carbonyl diimidazole as disclosed in Ann., 648, 72, (1961). Urea 7 may be obtained via the reaction of isocyanate 4 with ammonium hydroxide or gaseous ammonia. These reactions are illustrated in Scheme VI below as follows:

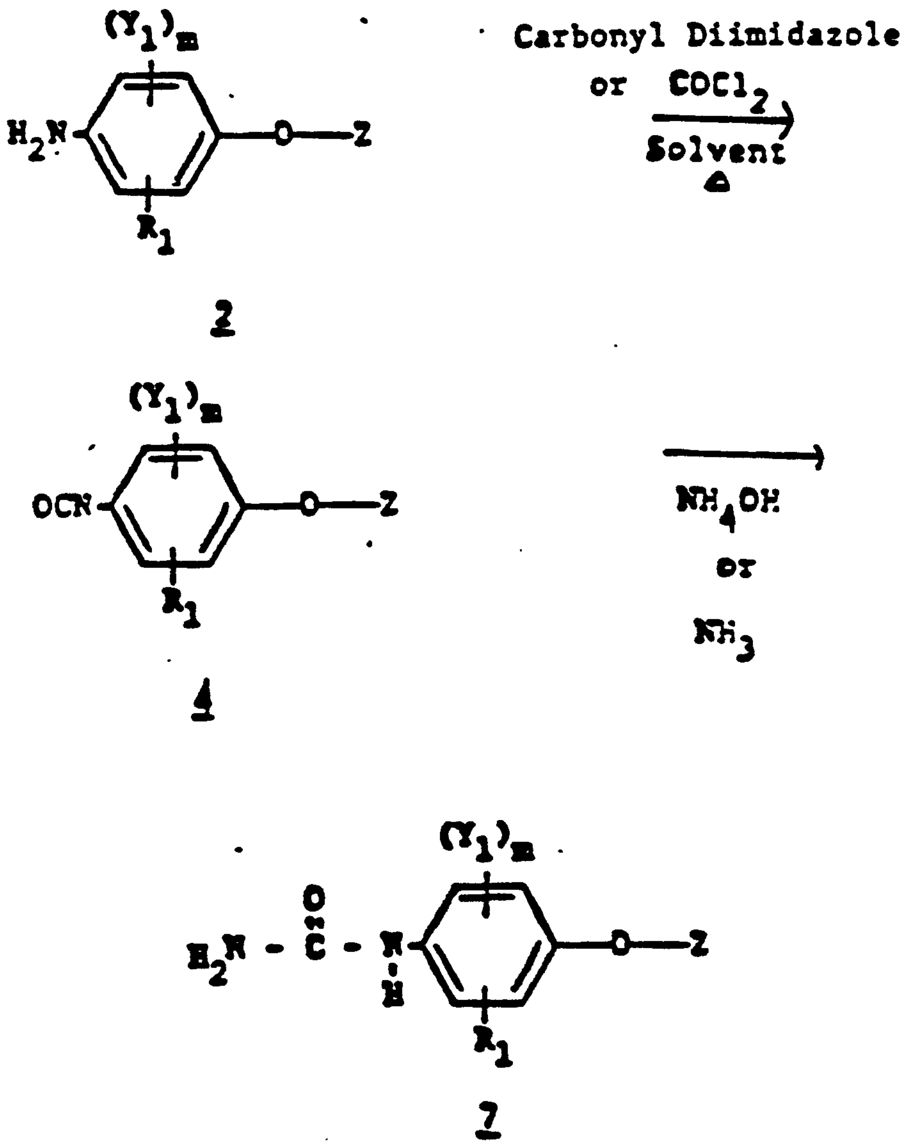


2

4

7

Scheme VI

wherein $Y_1$, m, $R_1$ and Z have the meaning given in Formula (1) except $R_1$ is not a carboxylic acid group or hydroxyalkyl group.

Substituted aryloxyanilines of type 2 can be obtained upon halogenation of aryloxyaniline 11 as depicted in Scheme VII below as follows:

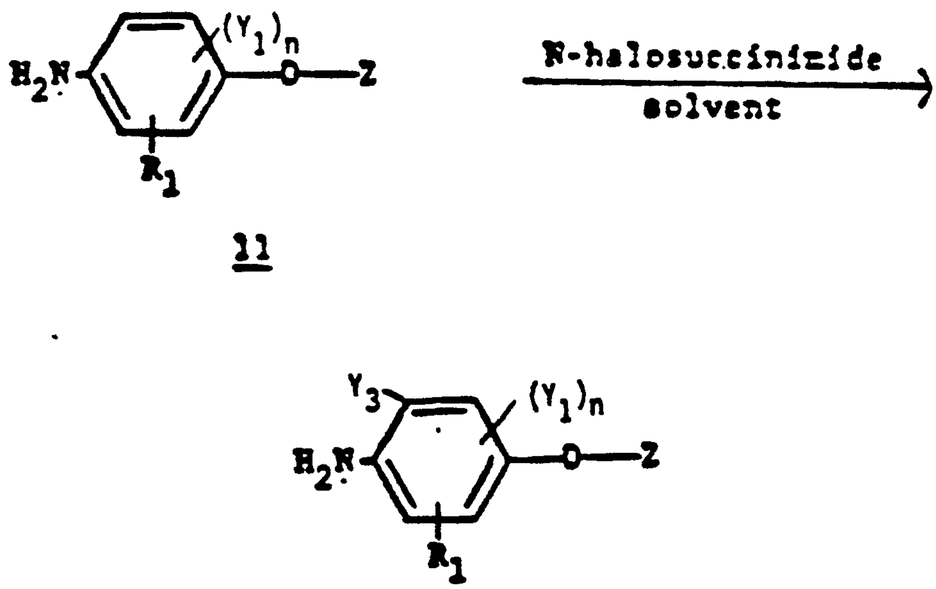


11

Scheme VII

wherein $Y_1$, $R_1$, and Z have the meaning given in Formula (1), n is a value of 0 or 1, and $Y_3$ is chloro or bromo. As is apparent from Scheme VII, at least one of the positions ortho to the amino group of aryloxyaniline 11 must be unsubstituted. Suitable solvents for this transformation include haloalkanes; aromatic hydrocarbons, such as benzene; or polar protic solvents, such as acetic acid. Halogenation of aryloxyaniline 11 may be effected by its exposure to chlorine or bromine in a suitable solvent at low temperature or preferably treatment with a N-halosuccinimide in benzene. Temperatures required for the reaction vary according to the identity of substituents $Y_1$ and $R_1$ but generally fall in the range of 25°C-80°C.

Aryloxyanilines of type 11 can be prepared by the method depicted in Scheme V above making use of chloronitrobenzene 12 as the electrophilic component in this coupling reaction.

Chloronitrobenzenes of type 12 are illustrated as follows:

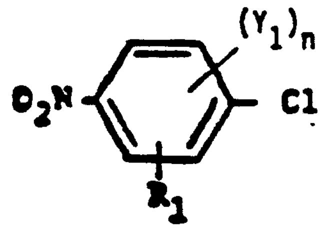

12

wherein $R_1$ has the meaning given in Formula (1) except $R_1$ is not an acyloxyalkyl group, $Y_1$ has the meaning given in formula (1) provided that when $Y_1$ is ortho to the nitro group then it is alkyl, and n has a value of 0 or 1.

The precursors to chloronitrobenzenes of type 8 (e.g. 16 below) can be obtained from the corresponding nitroaniline via the Sandmeyer procedure as practiced by Miller et. al. J. Med. Chem. 23, 1083 (1980).

Alternatively, chloronitrobenzenes of type 16 can be prepared through the nitration of a chlorobenzene as typified by the synthesis of chloronitrobenzene 16 illustrated in Scheme VIII as follows:

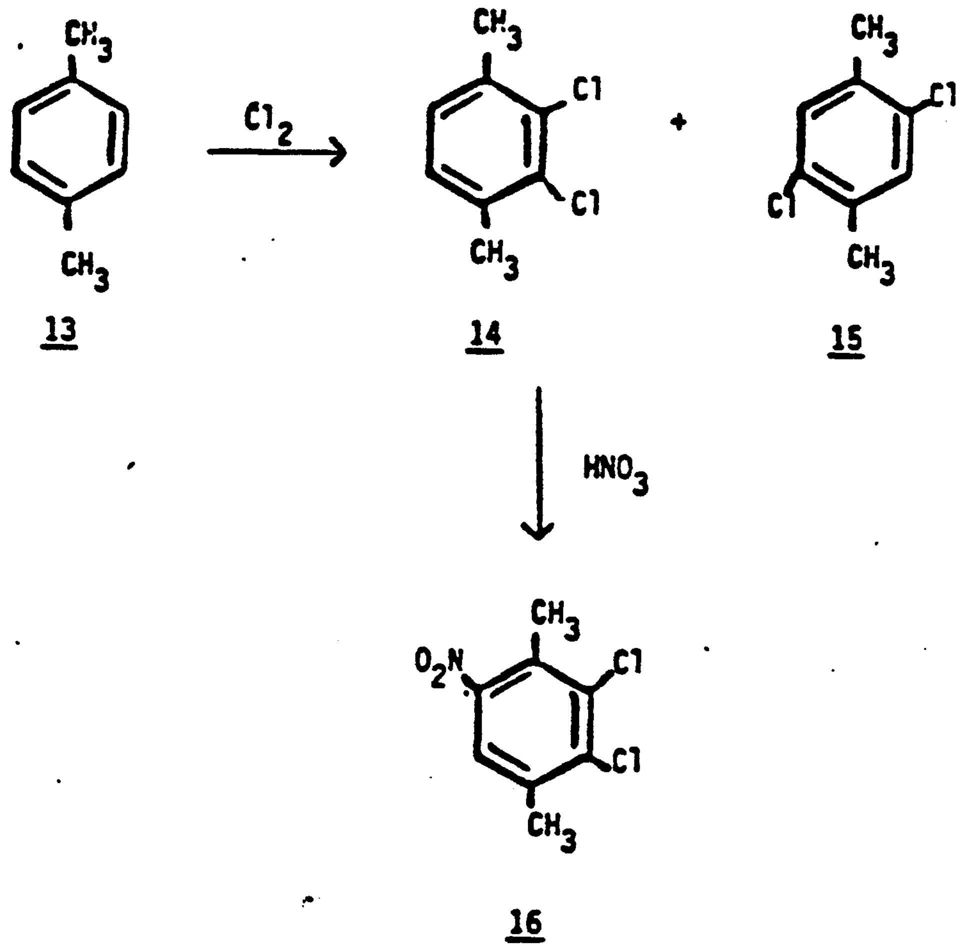


13 14 + 15

16

**Scheme VIII**

The 5-methyl group of chloronitrobenzene 16 may be elaborated to an acetal moiety according to Scheme IX as follows:

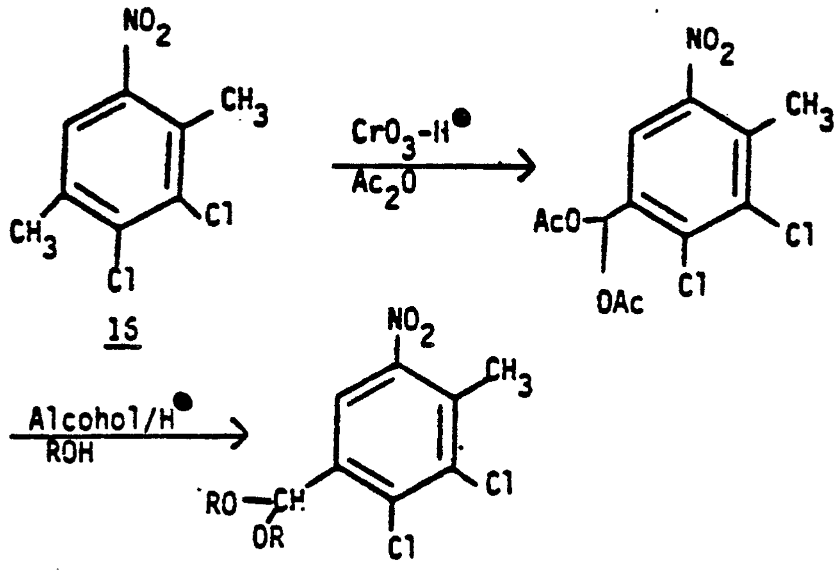


16

Scheme IX

wherein R can be alkyl, alkenyl, alkynyl, polyhaloalkyl or polyhaloalkenyl. Thus, chloronitrotoluene 16 can be treated with chromium trioxide in the presence of acetic anhydride and an acid catalyst to afford a masked aldehyde (Org. Synth . IV, 713). Exposure of the masked aldehyde to an alcohol in the presence of a catalytic amount of acid affords an illustrative functionalized chloronitrobenzene of type 8.

The preparation of aromatic alcohols of type 9 is illustrated by the elaboration of 4-bromo-2-fluorophenol from 2-fluorophenol using the procedure set forth by Mitchell et al., J. Org. Chem., 25, 4733 - (1979). Another phenolic intermediate, 2-bromo-4-chlorophenol, is obtained analogously from 4-chlorophenol.

An alternate route to aryloxyanilines of type 2 , in particular the aryloxyaniline 21, is depicted in Scheme X below as follows:

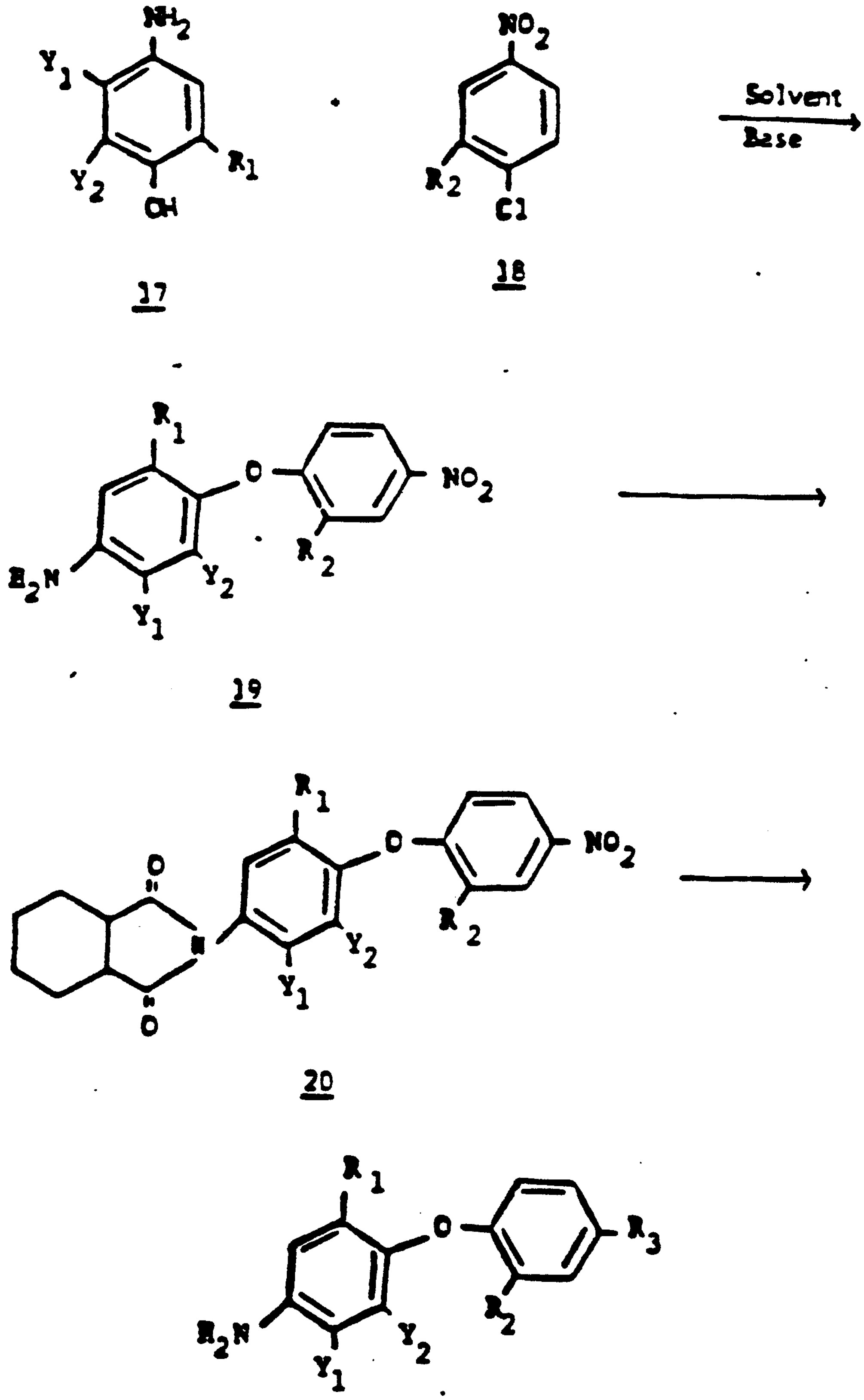


17

18

19

20

21

Scheme X

wherein $Y_1$ and $R_1$ have the meaning given in Formula (1) except $R_1$ is not an acyclic or cyclic acetal group or a carboxylic acid group, $Y_2$ independently has the meaning given for $Y_1$, $R_2$ is hydrogen, halogen, alkyl, haloalkyl or polyhaloalkyl and $R_3$ is hydrogen or halogen. This reaction involves the coupling of an aminophenol 17 with a 4-chloronitrobenzene 18 in the presence of a base to afford 4-nitrophenoxyaniline 19 as described in Schramm et al., Ann., 740, 169 (1970). Reaction of the amino group in 19 with cyclohexane -1,2-dicarboxylic anhydride affords imide 20. Nitro group reduction, Sandmeyer halogenation and deprotection of the amino function afford aniline 21 wherein $R_3$ is halogen. Reduction of the nitro group in imide 20 followed by diazotization and reduction of the diazonium salt (C.F. J. Am. Chem. Soc., (1952), 74, 3074 or Org. React. , (1944), 2, 262) affords aniline 21 wherein $R_3$ is hydrogen.

Aminophenols of type 17are readily available and may be prepared as illustrated in the elaboration of aminophenol 25 via nitration of a 2, 5-disubstituted phenol 22 followed by halogenation and nitro group reduction as depicted in Scheme XI below as follows:

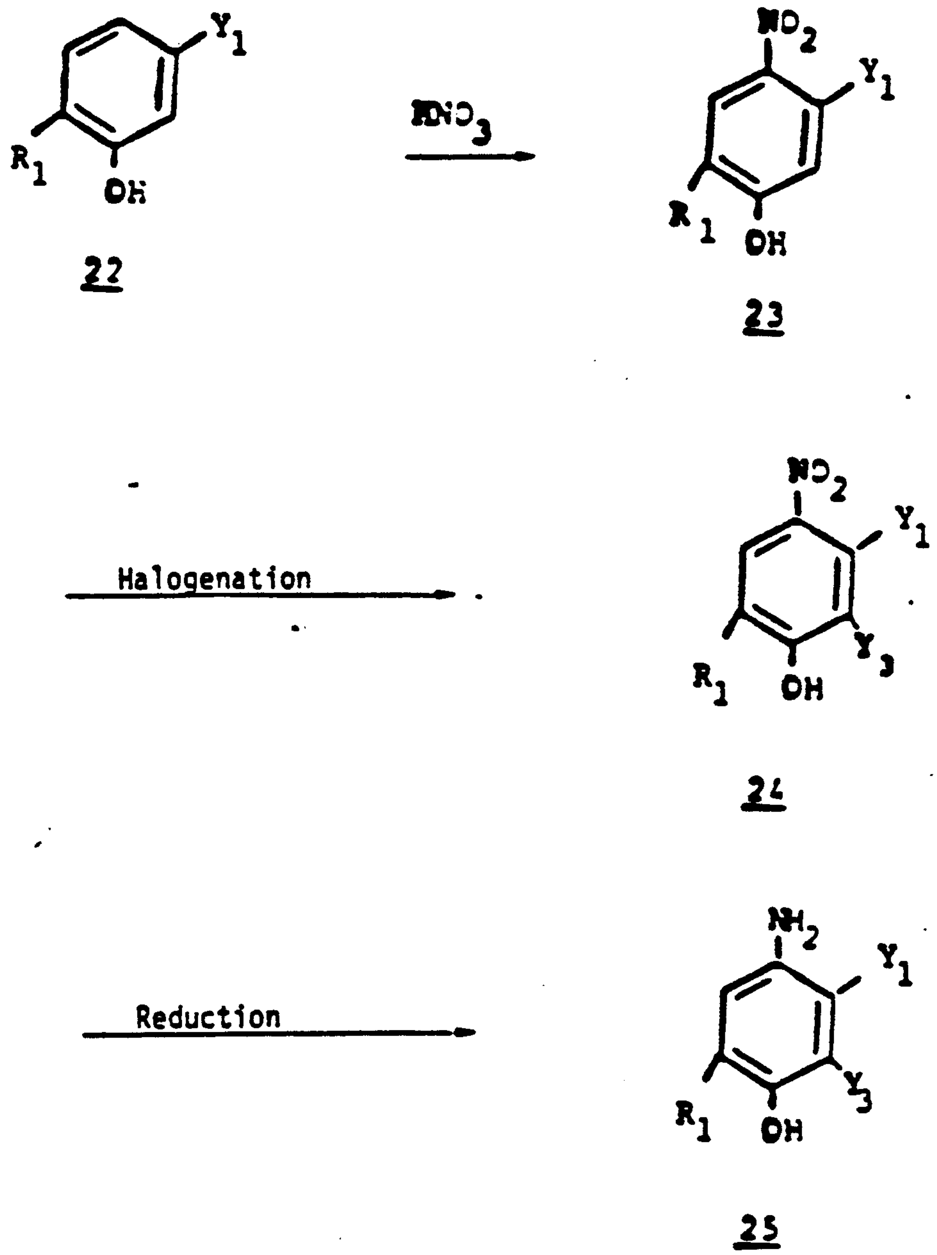


22

23

24

25

Scheme XI

wherein $Y_1$ and $R_1$ have the meaning given in Formula (1) except $R_1$ is not an acyclic or cyclic acetal group, an acyclic or cyclic dithioacetal group, an acyclic or cyclic hemithioacetal group, or a carboxylic acid group, and $Y_3$ is bromine or chlorine. This approach to intermediates 23 and 24reflects that described by Albert and Sears, J. Am. Chem. Soc., 76, 4979 (1954).

An alternate and complimentary approach to aminophenols 17 is detailed in Scheme XII as follows:

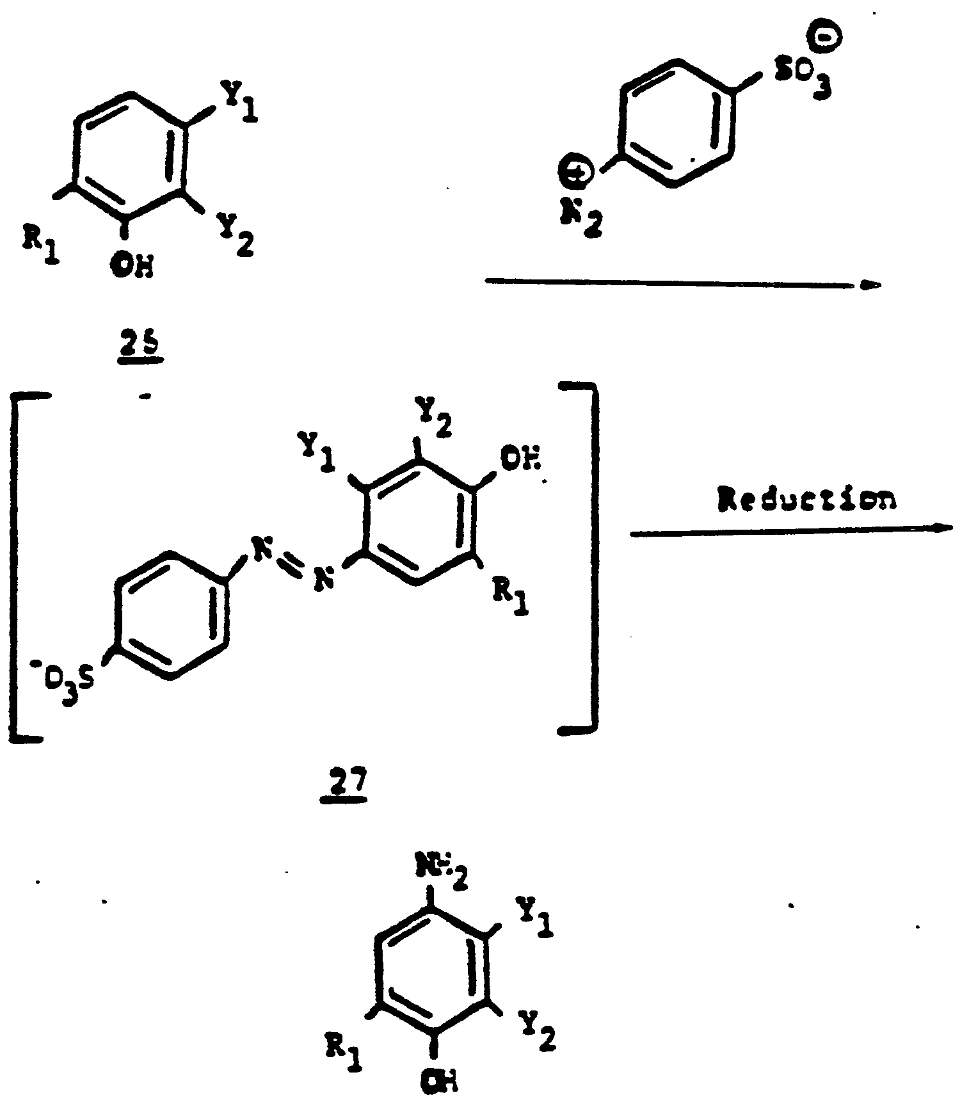


26

27

## Scheme XII

wherein $Y_1$ and $R_1$ have the meaning given in Formula (1), and $Y_2$ independently has the meaning given for $Y_1$. This involves the reaction of a trisubstituted phenol 26 with a diazonium salt prepared from sulfanilic acid to afford the intermediate diazo compound 27 which is reduced to give aminophenol 17. The synthetic methodology used in this approach to aminophenol 17 is that described by Payne and Weiden in U.S. Patent 3,752,838.

An alternate route to aryloxyphenyl benzoyl ureas encompassed by Formula (1) involves functional group manipulation of an aryloxynitrobenzene containing an acetal moiety (Scheme XIII) prior to nitro group reduction and coupling with a benzoyl isocyanate 3.

Thus, an acetal can undergo transacetalization upon exposure to an alcohol in the presence of a catalytic amount of acid to afford an aryloxynitro acetal. Nitro group reduction and exposure of the resultant aniline to benzoyl isocyanate 3 affords an aryloxyphenyl benzoyl urea acetal.

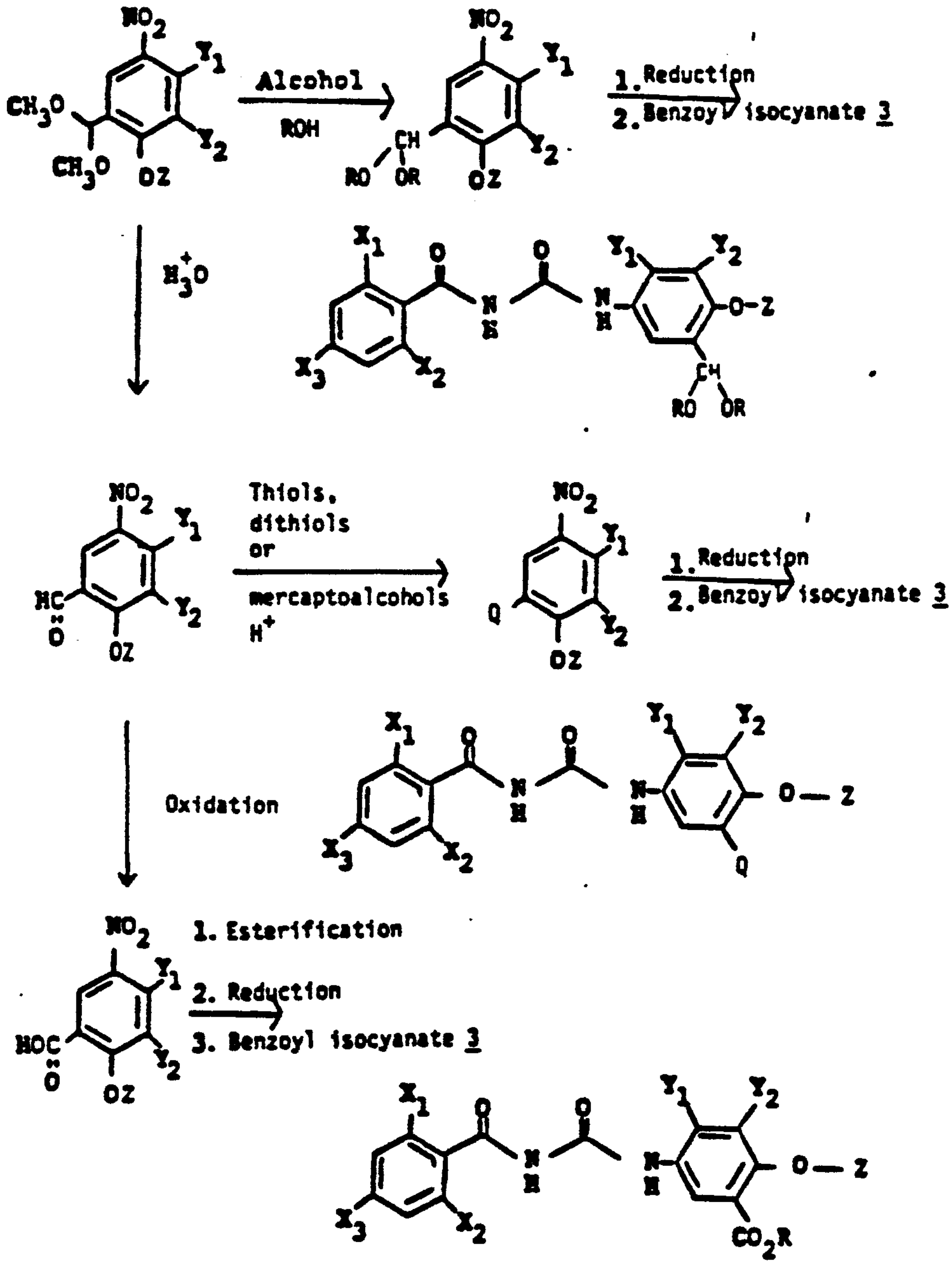


Scheme XIII

wherein $X_1$, $X_2$, $X_3$, $Y_1$ and Z have the meaning described in Formula (1), $Y_2$ independently has the meaning given for $Y_1$, R can be alkyl, alkenyl, alkynyl, polyhaloalkyl or polyhaloalkenyl, and Q can be an acyclic dithioacetal, a cyclic dithioacetal or a cyclic hemithioacetal moiety.

Hydrolysis of an acetal to the corresponding aldehyde proceeds in aqueous acid. Reaction of this aldehyde with thiols, dithiols or mercaptoalcohols in the presence of an acid catalyst affords the corresponding nitroethers bearing an acyclic dithioacetal, a cyclic dithioacetal or a cyclic hemithioacetal moiety respectively. Chemical reduction of the nitro group affords the corresponding aniline which, when reacted with a substituted benzoyl isocyanate, affords the functionalized benzoyl urea. Alternatively, oxidation of the aldehyde moiety to a carboxylic acid derivative, the subsequent esterification, nitro group reduction and coupling with a benzoyl isocyanate of which affords a benzoyl urea bearing an ester moiety on the linking ring.

The 1-(4-aryloxyphenyl)-3-benzoyl urea acetals described by this invention display not only outstanding characteristics as pesticides but may also serve as pivotal intermediates in the synthesis of a variety of other novel pesticidal compounds as illustrated below.

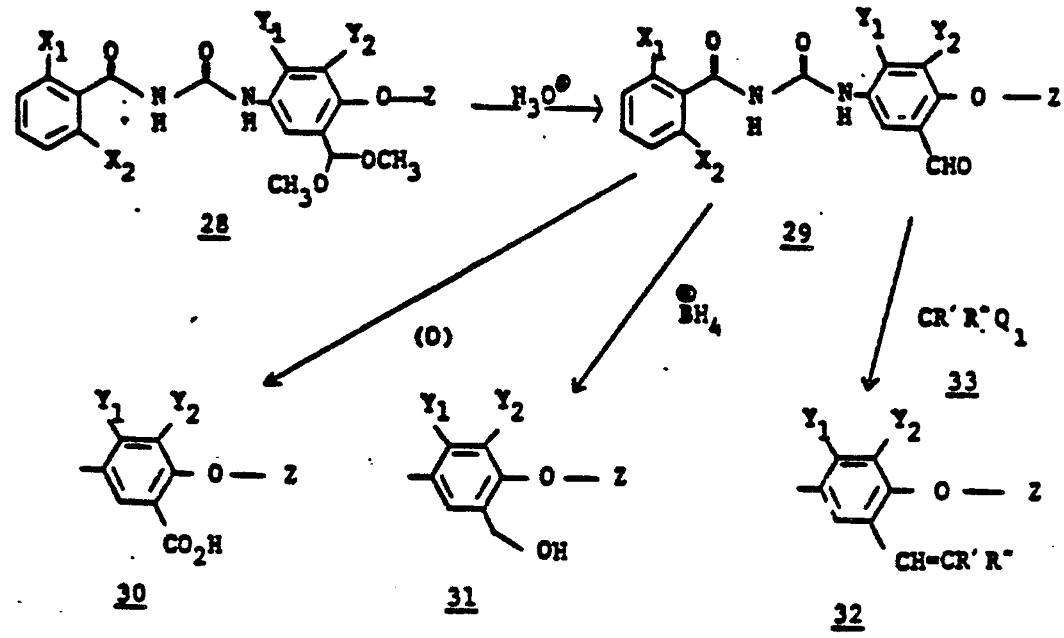


wherein $X_1$, $X_2$, $Y_1$ and Z have the meaning given in Formula (1); $Y_2$ independently has the meaning given for $Y_1$; R' and R" are independently hydrogen, alkyl, halogen, nitro, cyano or a carboxylic acid ester group, and $Q_1$ is $(EtO)_2PO$, $Ph_3P$ or hydrogen.

Mild acid hydrolysis of acetal 28 affords aldehyde 29 which upon exposure to an oxidant such as Jones Reagent affords acids of type 30. Borohydride reduction of 29 affords the hydroxymethyl compound 31 and reactions involving 29 and a reagent 33 afford olefins of type 32.

The compounds contemplated in this invention may be employed as insecticides according to methods known to those skilled in the art. Pesticidal compositions containing the compounds as the active toxicant will usually comprise a carrier and/or diluent, either liquid or solid.

Suitable liquid diluents or carriers include water, petrolium distillates, or other liquid carriers with or without surface active agents. Liquid concentrates may be prepared by dissolving one of these compounds with a nonphytotoxic solvent such as acetone, xylene, nitrobenzene, cyclohexanone or dimethyl formamide and dispersing the toxicants in water with the aid of suitable surface active emulsifying and dispersing agents.

The choice of dispersing and emulsifying agents and the amount employed is dictated by the nature of the composition and the ability of the agent to facilitate the dispersion of the toxicant. Generally, it is desirable to use as little of the agent as is possible, consistent with the desired dispersion of the toxicant in the spray so that rain does not re-emulsify the toxicant after it is applied to the plant and wash it off the plant. Nonionic, anionic, or cationic dispersing and emulsifying agents may be employed, for example, the condensation products of alkylene oxides with phenol and organic acids, alkyl aryl sulfonates, complex ether alcohols, quaternary ammonium compounds, and the like.

In the preparation of wettable powder or dust or granulated compositions, the active ingredient is dispersed in and on an appropriately divided solid carrier such as clay, talc, bentonite, diatomaceous earth, fullers earth, and the like. In the formulation of the wettable powders the aforementioned dispersing agents as well as lignosulfonates can be included.

The required amount of the toxicants contemplated herein may be applied per acre treated in from 1 to 200 gallons or more of liquid carrier and/or diluent or in from about 5 to 500 pounds of inert solid carrier and/or diluent. The concentration in the liquid concentrate will usually vary from about 10 to 95 percent by weight and in the solid formulations from about 0.5 to about 90 percent by weight. Satisfactory sprays, dusts, or granules for general use contain from about 1/4 to 15 pounds of active toxicant per acre.

The pesticides contemplated herein prevent attack by insects upon plants or other material to which the pesticides are applied, and they have relatively high residual toxicity. With respect to plants, they have a high margin of safety in that when used in sufficient amount to kill or repel the insects, they do not burn or injure the plant, and they resist weathering which includes wash-off caused by rain, decomposition by ultraviolet light, oxidation, or hydrolysis in the presence of moisture or, at least, such decomposition,

oxidation, and hydrolysis as would materially decrease the desirable pesticidal characteristic of the toxicants or impart undesirable characteristics, for instance, phytotoxicity, to the toxicants. The toxicants are so chemically inert that they are now compatible with substantially any other constituents of the spray - schedule, and they may be used in the soil, upon the seed, or the roots of plants without injuring either the seeds or roots of plants. Mixtures of the active compounds may be employed if desired as well as combinations of the active compounds of this invention with other biologically active compounds or ingredients.

The following examples are illustrative of the methods utilized in the preparation of intermediates and compounds of this invention. For NMR spectroscopic analysis, chemical shifts are reported in parts per million downfield from TMS.

Example A

Preparation of 5-bisacetoxymethyl-3,4-dichloro-2-methyl-1-nitrobenzene

Into a 5 liter, 3-necked reaction flask equipped with an internal thermometer and mechanical stirrer was charged 3,4-dichloro-2,5-dimethyl-1-nitrobenzene (200 grams, 0.909 moles). Acetic anhydride (1.0 liter) was then added. Complete dissolution of the solid nitrobenzene starting material was affected with stirring and gentle warming with a heat gun. The mixture was then chilled to a temperature of 0-10°C and concentrated sulfuric acid (200 milliliters) was added dropwise. A solution of chromium trioxide was then prepared as follows: chromium trioxide (245.6 grams, 2.46 moles) was added portionwise with stirring to 1.0 liter of acetic anhydride contained in an Erlenmeyer flask at ambient temperature. This solution was then added dropwise over a 2-hour period to the paste-like reaction mixture while maintaining the temperature below 10°C. The mixture was then allowed to warm to room temperature and stirred for 1.5 hours. The mixture was poured into chilled water (16 liters) and allowed to stand overnight. The water was decanted off and the residue was dissolved in ethyl acetate. The organic layer was washed with water, saturated sodium bicarbonate and brine solutions; dried over sodium sulfate, filtered and concentrated under reduced pressure to afford 200 grams (65%) of a brown oil. Recrystallization from ethyl acetate; hexane afforded 5-bisacetoxymethyl-3,4-dichloro-2-methyl-1-nitrobenzene (47.9 grams, 0.142 moles, 16%) as a cream colored powder having a melting point of 99-107°C. Elemental analysis of the cream colored powder indicated the following:

Analysis: $C_{12}H_{11}Cl_2NO_6$

Calculated: C, 42.88; H, 3.30; N, 4.17

Found: C, 42.62; H, 3.49; N, 4.04

Example B

Preparation of 3,4-dichloro-5-dimethoxymethyl-2-methyl-1-nitrobenzene

To a solution of 5-bisacetoxymethyl-3,4-dicholoro-2-methyl-1-nitrobenzene (47.9 grams, 0.143 moles) prepared in Example A in methanol (1.4 liters) was added concentrated sulfuric acid (10 drops). The solution was heated overnight at reflux, cooled, quenched with saturated sodium bicarbonate solution and concentrated under reduced pressure. When most of the methanol had been removed, ethyl acetate was added and the organic layer was washed with water and brine, dried over sodium sulfate and concentrated under reduced pressure to afford 3,4-dichloro-5-dimethoxymethyl-1-nitrobenzene (38.7 grams, 0.138 mmol, 97%) as a brown oil. Elemental analysis of the brown oil indicated the following:

Analysis: $C_{10}H_{11}Cl_2NO_4$

Calculated: C, 42.88; H, 3.96; N, 5.00

Found: C, 43.07; H, 3.93, N, 5.36

Example C

Preparation of 4-(2-bromo-4-chlorophenoxy)-3-chloro-5-dimethoxymethyl-2-methyl-1-nitrobenzene

Into a 300 milliliter, 3-necked round-bottom reaction flask equipped with a condenser, nitrogen inlet and magnetic stirrer was charged 3,4-dichloro-5-dimethoxymethyl-2-methyl-1-nitrobenzene prepared in Example B (7.50 grams, 26.78 mmol), 2-bromo-4-chlorophenol (6.67 grams, 32.14 mmol), potassium carbonate (7.40 grams, 53.56 mmol), and dimethylformamide (60 milliliters) and the mixture was heated overnight at a temperature of 110°C. The mixture was allowed to cool, transferred to a 1 liter round-bottomed flask and most of the dimethylformamide was distilled off under reduced pressure. Toluene and 5% sodium hydroxide solution were then added. The organic layer was separated, washed successively with 5% sodium hydroxide, water and brine; dried over magnesium sulfate and concentrated under reduced pressure to afford a brown solid. Recrystallization from hot methylene chloride-methanol afforded 4-(2-bromo-4-chlorophenoxy)-3-chloro-5-dimethoxymethyl-2-methyl-1-nitrobenzene (4.78 grams, 10.60 mmol, 40%) as a crystalline powder having a melting point of 116-117°C. Elemental analysis of the crystalline powder indicated the following:
Analysis: $C_{16}H_{14}Cl_2BrNO_5$
Calculated: C, 42.60; H, 3.13; N, 3.11
Found: C, 42.93; H, 3.22; N, 3.05

Example D

Preparation of 4-(2-bromo-4-chlorophenoxy)-3-chloro-5-dimethoxymethyl-2-methylaniline

A Parr bottle was charged with 4-(2-bromo-4-chlorophenoxy)-3-chloro-5-dimethoxymethyl-2-methyl-1-nitrobenzene prepared in Example C (4.18 grams, 9.27 mmol) and a mixture of 5:1 toluene: ethyl acetate - (120 milliliters). The reaction vessel was then purged thoroughly with nitrogen and 5% platinum on carbon - (0.5 grams) was added immediately following the purge. The mixture was hydrogenated at 40-50 psi with agitation for a period of 1.5 hours. The mixture was then filtered through a pad of celite and concentrated under reduced pressure to afford 4-(2-bromo-4-chlorophenoxy)-3-chloro-5-dimethoxymethyl-2-methylaniline in quantitative yield having a melting point of 65-71°C. Elemental analysis indicated the following:
Analysis: $C_{16}H_{16}BrCl_2NO_3$
Calculated: C, 45.64; H, 3.83; N, 3.33
Found: C, 45.94; H, 3.91; N, 3.28

Example E

Preparation of 3-chloro-4-(2,4-difluorophenoxy)-5-formyl-2-methylnitrobenzene

Into a 500 milliliter round-bottom flask equipped with a magnetic stirrer, heating mantle, reflux condenser and nitrogen inlet was charged 3-chloro-4-(2,4-difluorophenoxy)-5-dimethoxymethyl-2-methyl-nitrobenzene (12.0 grams, 32.11 mmol). Acetic acid (128 milliliters) was then added with stirring, followed by addition of water (32 milliliters). The heterogeneous mixture was heated to reflux for one hour and poured into water (500 milliliters). The mixture was extracted with toluene and the organic layer was washed with saturated sodium bicarbonate and brine solutions, dried over sodium sulfate, filtered and concentrated under reduced pressure to afford 3-chloro-4-(2,4-difluorophenoxy)-5-formyl-2-methylnitrobenzene (10.1 grams, 30.82 mmol, 96.9%) as a yellow solid having a melting point of 150-153°C. Elemental analysis of the yellow solid indicated the following:
Analysis: $C_{14}H_8ClF_2NO_4$
Calculated: C, 51.32; H, 2.46; N, 4.27
Found: C, 51.31; H, 2.47; N, 4.19

Example F

Preparation of 2-(3-chloro-2-(2,4-difluorophenoxy)-4-methyl-5-nitrophenyl)-1,3-dithiolane

Into a 250 milliliter round-bottom reaction flask equipped a magnetic stirrer, heating mantle, reflux condenser, nitrogen inlet and gas exit tube attached to a trap containing 10% aqueous sodium hydroxide was added 3-chloro-4-(2,4-difluorophenoxy)-5-formyl-2-methyl-1-nitrobenzene (7.0 grams, 21.36 mmoles) as a slurry in toluene (43 milliliters). Ethanedithiol (2.21 grams, 24.0 mmoles) and p-toluenesulfonic acid monohydrate (0.4 grams, 2.1 mmol) were then added and the mixture heated to reflux for 30 minutes. The reaction vessel was then fitted with a Dean-Stark trap and reflux was continued for an additional 35 minutes. The mixture was allowed to stand overnight at room temperature under a nitrogen atmosphere, diluted with toluene and washed with saturated sodium bicarbonate and brine solutions. The organic layer was dried over sodium sulfate, filtered and concentrated under reduced pressure to afford 2-(3-chloro-2-(2,4-difluorophenoxy)-4-methyl-5-nitrophenyl)-1,3-dithiolane (8.30 grams, 20.55 mmol, 96.2%) as a pale yellow solid having a melting point of 114-117°C. Elemental analysis of the yellow solid indicated the following:
Analysis: $C_{16}H_{12}ClF_2NO_3S_2$
Calculated: C, 47.58; H, 3.00; N, 3.47
Found: C, 47.72: H, 2.97; N, 3.39

Example G

Preparation of 2-(5-amino-3-chloro-2-(2,4-difluorophenoxy)-4-methylphenyl)-1,3-dithiolane

Into a 100 milliliter round-bottom flask equipped with a magnetic stirrer, heating mantle, reflux condenser, nitrogen inlet, thermometer and gas exit tube attached to a trap containing 10% aqueous sodium hydroxide was added 2-(3-chloro-2-(2,4-difluorophenoxy)-4-methyl-5-nitrophenyl)-1,3-dithiolane (8.0 grams, 19.81 mmol) as a slurry in ethanol (40 milliliters). Tin (II) chloride dihydrate (22.35 grams, 99.05 mmol) was added and the mixture heated at a temperature of 62-78°C for one hour. The mixture was allowed to cool to room temperature then poured into ice water (400 milliliters). After the ice had melted, the pH of the mixture was adjusted to pH 7 with 5% aqueous sodium bicarbonate. The mixture was extracted - (2X) with ethyl acetate. The organic layer was washed with brine, dried over sodium sulfate, filtered and concentrated under reduced pressure to afford 2-(5-amino-3-chloro-2-(2,4-difluorophenoxy)-4-methylphenyl)-1,3-dithiolane (6.87 grams, 18.38 mmol, 92.7%) as a viscous oil. $^1$H-NMR analysis indicated the following: $^1$H-NMR ($CDCl_3$): $\delta$ 7.43-6.30 (m, 3H), 6.22 (s, 1H), 5.85 (s, 1H), 3.80 (br s, 2H), 3.72-3.06 (m, 4H), 2.24 (s, 3H).

Example 1

Preparation of 1-(3-chloro-4-(2,4-dichlorophenoxy-5-dimethoxymethyl-2-methylphenyl)-3-(2,6-difluorobenzoyl) urea

Into a round-bottom reaction flask was charged 3-chloro-4-(2,4-dichlorophenoxy)-5-dimethoxymethyl-2-methylaniline (8.90 grams, 23.63 mmol) prepared in a manner similar to Example D and toluene (45 milliliters). The mixture was warmed to effect complete disolution of the aniline and then purged with nitrogen. Neat 2,6-difluorobenzoyl isocyanate (4.33 grams, 23.63 mmol) was then added via syringe to the reaction mixture. A mild exotherm was accompanied by an almost immediate precipitation of the product benzoyl urea. The product was filtered and washed with cold ether to afford 1-(3-chloro-4-(2,4-dichlorophenoxy)-5-dimethoxymethyl-2-methylphenyl-3-(2,6-difluorobenzoyl) urea (11.90 grams, 21.26 mmol, 90%) as a crystalline powder having a melting point of 190-191°C. Elemental analysis of the crystalline powder indicated the following:
Analysis: $C_{24}H_{19}Cl_3F_2N_2O_5$
Calculated: C, 51.50; H, 3.42; N, 5.01
Found: C, 51.45; H, 3.55; N, 4.90

Example 2

Preparation of 1-(3-chloro-4-(2,4-dichlorophenoxy)-5-formyl-2-methylphenyl)-3-(2,6-difluorobenzoyl) urea

Into a round-bottom reaction flask equipped with a magnetic stirrer and reflux condenser was added 1-(3-chloro-4-(2,4-dichlorophenoxy)-5-dimethoxymethyl-2-methylphenyl)-3-(2,6-difluorobenzoyl) urea (10.6 grams, 18.94 mmol) prepared in Example 1 and a 3:1 mixture of acetic acid: water (200 milliliters). The heterogeneous mixture was heated to reflux for 1 hour and then cooled to room temperature. Water (200 milliliters) was added to the reaction mixture and the product was filtered, washed with water and cold methanol and dried in a vacuum oven to afford 1-(3-chloro-4-(2,4-dichlorophenoxy)-5-formyl-2-methylphenyl)-3-(2,6-difluorobenzoyl) urea (9.00 grams, 17.52 mmol, 92%) as a white powder having a melting point of 222-225°C. Elemental analysis of the white powder indicated the following:
Analysis: $C_{22}H_{13}Cl_3F_2N_2O_4$
Calculated: C, 51.44; H, 2.55; N, 5.45
Found: C, 51.57; H, 2.54; N, 5.61

EXAMPLE 3

Preparation of 1-(3-chloro-4-(2,4-dichlorophenoxy)-5-hydroxymethyl-2-methylphenyl)-3-(2,6-difluorobenzoyl) urea

To a solution of 1-(3-chloro-4-(2,4-dichlorophenoxy)-5-formyl-2-methylphenyl)-3-(2,6-difluorobenzoyl) urea (2.00 grams, 3.89 mmol) prepared in Example 2 in methanol (48 milliliters) was added sodium borohydride (150 milligrams, 3.89 mmol) as a powder. Tetrahydrofuran (20 milliliters) was then added and the reaction mixture stirred overnight at ambient temperature. Additional sodium borohydride (150 milligrams, 3.89 mmol) was then added and the reaction mixture stirred for 0.5 hours. Excess sodium borohydride was destroyed and the product precipitated with the addition of 5% sulfuric acid (100 milliliters). The precipitated product was collected on a fritted funnel, washed with water and ether to give 1-(3-chloro-4-(2,4-dichlorophenoxy)-5-hydroxymethyl-2-methylphenyl)-3-(2,6-difluorobenzoyl) urea (750 milligrams, 1.45 mmol, 37%) having a melting point of 196-198°C. Elemental analysis indicated the following:
Analysis: $C_{22}H_{15}Cl_3F_2N_2O_4$
Calculated: C, 51.24; ;H, 2.93; N, 5.43
Found: C, 51.02; H, 3.00; N, 4.97

EXAMPLE 4

Preparation of 1-(5-carboxy-3-chloro-4-(2,4-dimethylphenoxy)-2-methylphenyl)-3-(2,6-difluorobenzoyl) urea

To a magnetically stirred solution of 1-(3-chloro-4-(2,4-dimethylphenoxy)-5-formyl-2-methylphenyl)-3-(2,6-difluorobenzoyl) urea (1.20 grams, 2.50 mmol) prepared in Example 2 in acetone (50 milliliters) was added 1.1 milliliters of Jones reagent. The mixture was stirred for a period of 1.5 hours followed by the addition of additional Jones reagent (1.0 milliliter). The mixture was stirred for 15 minutes, quenched with saturated sodium sulfite solution (5 milliliters) and concentrated under reduced pressure. Ethyl acetate was added to the residue and the organic layer was filtered, washed with water and brine, dried over sodium sulfate and concentrated under reduced pressure to afford the crude product as a highly insoluble white powder. Trituration of this material with 1:1 hexane:ethyl acetate afforded 1-(5-carboxy-3-chloro-4-(2,4-dimethylphenoxy)-2-methylphenyl)-3-(2,6-difluorobenzoyl) urea (560 milligrams, 1.14 mmol, 46%) having a melting point of 237-238°C. Elemental analysis indicated the following:
Analysis: $C_{24}H_{19}ClF_2N_2O_5$
Calculated: C, 58.97; H, 3.92; N, 5.73
Found: C, 58.73; H, 3.94; N, 5.58

EXAMPLE 5

Preparation of 1-(3-chloro-4-(2,4-dimethylphenoxy)-5-(2-methoxycarbonyl-1-ethenyl)-2-methylphenyl)-3-(2,6-difluorobenzoyl) urea

Into a 100 milliliter round bottom reaction flask equipped with a magnetic stirrer was added 1-(3-chloro-4-(2,4-dimethylphenoxy)-5-formyl-2-methylphenyl)-3-(2,6-difluorobenzoyl) urea (2.30 grams, 4.86 mmol) prepared as in Example 2, (2-methoxy-2-oxoethyl)triphenylphosphonium bromide (2.22 grams, 5.34 mmol) and tetrahydrofuran (20 milliliters). To this heterogeneous mixture was added 1,8-diazabicyclo[5.4.0]undec-7-ene (DBU) (763 milligrams, 5.01 mmol). The mixture was stirred for a period of 3.5 hours, filtered and concentrated under reduced pressure. Flash column chromatography (3:1 hexane:ethyl acetate) afforded 1-(3-chloro-4-(2,4-dimethylphenoxy)-5-(2-methoxycarbonyl-1-ethenyl)-2-methylphenyl)-3-(2,6-difluorobenzoyl) urea (1.5 grams, 2.84 mmol, 58%) having a melting point of 198-205°C. Elemental analysis indicated the following:

Analysis: $C_{27}H_{23}ClF_2N_2O_5$
Calculated: C, 61.31, H, 4.38: N, 5.30
Found: C, 61.25; H, 4.38; N, 5.14

EXAMPLES 6 through 38

In a manner similar to that employed in the preceding examples, and using one of the synthesis - schemes previously disclosed, other urea compounds were prepared. The identity of the substituents on the generic formula and the analytical data are set forth in Tables I and II below:

**Table 1**

**Representative 1-(4-Phenoxyphenyl)-3-Benzoyl Urea Compounds**

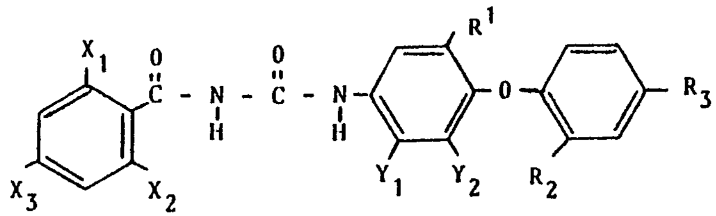

| Example | Molecular Formula | $X_1$ | $X_2$ | $X_3$ | $Y_1$ | $Y_2$ | $R_1$ | $R_2$ | $R_3$ | Elemental Analysis Calculated C | H | N | Found C | H | N | Melting Point °C |
|---|---|---|---|---|---|---|---|---|---|---|---|---|---|---|---|---|
| 6 | $C_{26}H_{25}ClF_2N_2O_5$ | F | F | H | $CH_3$ | Cl | $CH(OCH_3)_2$ | $CH_3$ | $CH_3$ | 60.18 | 4.86 | 5.40 | 60.00 | 5.00 | 5.26 | 175-177 |
| 7 | $C_{24}H_{20}Cl_4N_2O_5$ | Cl | H | H | $CH_3$ | Cl | $CH(OCH_3)_2$ | Cl | Cl | 51.64 | 3.61 | 5.02 | 51.42 | 3.56 | 4.92 | 163-166 |
| 8 | $C_{24}H_{19}BrCl_2F_2N_2O_5$ | F | F | H | $CH_3$ | Cl | $CH(OCH_3)_2$ | Br | Cl | 47.71 | 3.17 | 4.64 | 48.14 | 3.18 | 4.56 | 184-186 |
| 9 | $C_{24}H_{20}BrCl_3N_2O_5$ | Cl | H | H | $CH_3$ | Cl | $CH(OCH_3)_2$ | Br | Cl | 47.83 | 3.35 | 4.65 | 48.32 | 3.41 | 4.60 | 162-164 |
| 10 | $C_{26}H_{23}Cl_3F_2N_2O_5$ | F | F | H | $CH_3$ | Cl | $CH(OCH_2CH_3)_2$ | Cl | Cl | *HRMS 586.0640 | | | *HRMS 586.0606 | | | 171-176 |
| 11 | $C_{23}H_{15}Cl_3F_2N_2O_5$ | F | F | H | $CH_3$ | Cl | $CO_2CH_3$ | Cl | Cl | 50.81 | 2.78 | 5.15 | 50.73 | 2.74 | 5.03 | 180-183 |
| 12 | $C_{24}H_{17}BrCl_2F_2N_2O_5$ | F | F | H | $CH_3$ | Cl | $CH_2(OCH_2)_2$ | Br | Cl | *HRMS 601.9647 | | | *HRMS 601.9670 | | | 170-173 |
| 13 | $C_{24}H_{18}BrCl_3N_2O_5$ | Cl | H | H | $CH_3$ | Cl | $CH_2(OCH_2)_2$ | Br | Cl | 47.99 | 3.02 | 4.66 | 48.15 | 3.06 | 4.57 | 169-171 |
| 14 | $C_{22}H_{13}BrCl_2F_2N_2O_4$ | F | F | H | $CH_3$ | Cl | CHO | Br | Cl | 47.34 | 2.35 | 5.02 | 47.06 | 2.32 | 4.71 | 213-214.5 |
| 15 | $C_{24}H_{18}BrCl_2FN_2O_5$ | F | H | H | $CH_3$ | Cl | $CH_2(OCH_2)_2$ | Br | Cl | 49.34 | 3.11 | 4.79 | 49.58 | 3.25 | 4.75 | 145.5-157 |

*High Resolution Mass Spectrum

**Table 1 Cont'd)**

**Representative 1-(4-Phenoxyphenyl)-3-Benzoyl Urea Compounds**

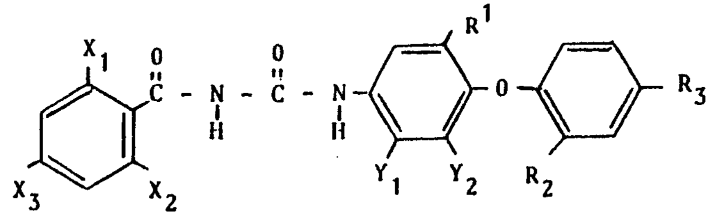

| Example | Molecular Formula | $X_1$ | $X_2$ | $X_3$ | $Y_1$ | $Y_2$ | $R_1$ | $R_2$ | $R_3$ | Elemental Analysis Calculated C | H | N | Found C | H | N | Melting Point °C |
|---|---|---|---|---|---|---|---|---|---|---|---|---|---|---|---|---|
| 16 | $C_{24}H_{17}Cl_3F_2N_2O_5$ | F | F | H | $CH_3$ | Cl | $CH_2(OCH_2)_2$ | Cl | Cl | 51.68 | 3.07 | 5.02 | 51.95 | 3.21 | 4.86 | 197-199 |
| 17 | $C_{24}H_{18}Cl_3FN_2O_5$ | F | H | H | $CH_3$ | Cl | $CH_2(OCH)_2)_2$ | Cl | Cl | 53.40 | 3.36 | 5.19 | 53.45 | 3.21 | 5.00 | 142-146 |
| 18 | $C_{24}H_{18}Cl_4N_2O_5$ | Cl | H | H | $CH_3$ | Cl | $CH_2(OCH)_2)_2$ | Cl | Cl | 51.82 | 3.26 | 5.04 | 52.02 | 3.24 | 4.97 | 167-169 |
| 19 | $C_{22}H_{15}BrCl_2F_2N_2O_4$ | F | F | H | $CH_3$ | Cl | $CH_2OH$ | Br | Cl | 47.16 | 2.69 | 5.00 | 47.30 | 2.62 | 5.10 | 177-180 |
| 20 | $C_{23}H_{17}Cl_3F_2N_2O_4$ | F | F | H | $CH_3$ | Cl | $CH_2OCH_3$ | Cl | Cl | 52.14 | 3.23 | 5.28 | 51.84 | 3.22 | 5.25 | 178-179 |
| 21 | $C_{23}H_{15}BrCl_2F_2N_2O_5$ | F | F | H | $CH_3$ | Cl | $CO_2CH_3$ | Br | Cl | *HRMS 585.9510 | | | *HRMS 585.9567 | | | 195.5-199.5 |
| 22 | $C_{23}H_{16}BrCl_3N_2O_5$ | Cl | H | H | $CH_3$ | Cl | $CO_2CH_3$ | Br | Cl | 47.08 | 2.74 | 4.77 | 47.07 | 2.72 | 4.63 | 173-175 |
| 23 | $C_{23}H_{16}BrCl_2FN_2O_5$ | F | H | H | $CH_3$ | Cl | $CO_2CH_3$ | Br | Cl | 48.44 | 2.82 | 4.91 | 48.84 | 2.73 | 4.79 | 197.5-203.5 |
| 24 | $C_{24}H_{17}BrCl_2F_2N_2O_5$ | F | F | H | $CH_3$ | Cl | $CO_2CH_2CH_3$ | Br | Cl | 47.86 | 2.84 | 4.65 | 48.31 | 2.92 | 4.53 | 198-199.5 |
| 25 | $C_{24}H_{18}BrCl_2FN_2O_5$ | F | H | H | $CH_3$ | Cl | $CO_2CH_2CH_3$ | Br | Cl | 49.33 | 3.10 | 4.79 | 49.10 | 3.52 | 4.62 | 160.5-162 |
| 26 | $C_{26}H_{22}BrCl_2FN_2O_5$ | F | H | H | $CH_3$ | Cl | $CO_2CH_2CH(CH_3)_2$ | Br | Cl | 51.00 | 3.62 | 4.57 | 51.03 | 3.62 | 4.53 | 150-153 |

*High Resolution Mass Spectrum

**Table 1 Cont'd)**

**Representative 1-(4-Phenoxyphenyl)-3-Benzoyl Urea Compounds**

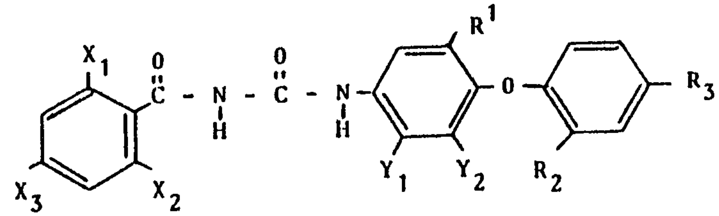

| Example | Molecular Formula | $X_1$ | $X_2$ | $X_3$ | $Y_1$ | $Y_2$ | $R_1$ | $R_2$ | $R_3$ | Elemental Analysis Calculated C | H | N | Found C | H | N | Melting Point °C |
|---|---|---|---|---|---|---|---|---|---|---|---|---|---|---|---|---|
| 27 | $C_{26}H_{21}BrCl_2F_2N_2O_5$ | F | F | H | $CH_3$ | Cl | $CO_2CH(CH_3)_2$ | Br | Cl | 49.54 | 3.35 | 4.44 | 49.57 | 3.35 | 4.40 | 175-179 |
| 28 | $C_{24}H_{17}Cl_3F_2N_2O_3S_2$ | F | F | H | $CH_3$ | Cl | $CH(SCH_2)_2$ | Cl | Cl | *HRMS 587.9714 | | | *HRMS 587.9718 | | | 176-180 |
| 29 | $C_{24}H_{18}Cl_4N_2O_3S_2$ | Cl | H | H | $CH_3$ | Cl | $CH(SCH_2)_2$ | Cl | Cl | 48.99 | 3.08 | 4.76 | 49.15 | 3.17 | 4.81 | 165-169 |
| 30 | $C_{24}H_{17}Cl_3F_2N_2O_4S$ | F | F | H | $CH_3$ | Cl | $CH(SCH_2CH_2O)$ | Cl | Cl | 50.23 | 2.99 | 4.88 | 50.13 | 2.99 | 4.78 | 176-180 |
| 31 | $C_{24}H_{18}Cl_4N_2O_4S$ | Cl | H | H | $CH_3$ | Cl | $CH(SCH_2CH_2O)$ | Cl | Cl | *HRMS 569.9741 | | | *HRMS 569.9706 | | | 110-112 |
| 32 | $C_{24}H_{17}ClF_4N_2O_3S_2$ | F | F | H | $CH_3$ | Cl | $CH(SCH_2)_2$ | F | F | 51.75 | 3.08 | 5.03 | 52.30 | 3.19 | 4.91 | 185-189 |
| 33 | $C_{24}H_{18}Cl_2F_2N_2O_3S_2$ | Cl | H | H | $CH_3$ | Cl | $CH(SCH_2)_2$ | F | F | 51.89 | 3.27 | 5.04 | 52.01 | 3.29 | 5.00 | 123-128 |

*High Resolution Mass Spectrum

**Table II**

**Representative 1-(4-Naphthoxyphenyl)-3-Benzoyl Urea Compounds**

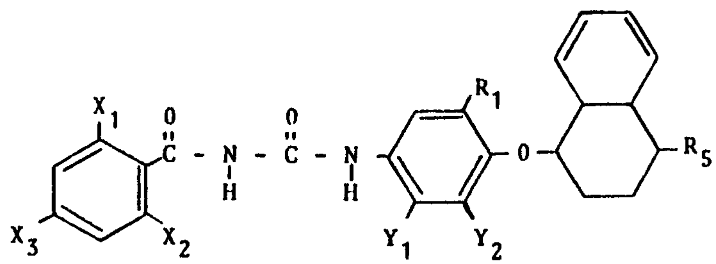

| Example | Molecular Formula | $X_1$ | $X_2$ | $X_3$ | $Y_1$ | $Y_2$ | | $R_5$ | Elemental Analysis Calculated C | H | N | Found C | H | N | Melting Point °C |
|---|---|---|---|---|---|---|---|---|---|---|---|---|---|---|---|
| 34 | $C_{28}H_{22}Cl_2F_2N_2O_5$ | F | F | H | $CH_3$ | Cl | $CH(OCH)_3)_2$ | Cl | 58.45 | 3.85 | 4.87 | 58.73 | 4.19 | 4.81 | 200-202 |
| 35 | $C_{27}H_{19}Cl_3N_2O_5$ | Cl | H | H | $CH_3$ | Cl | $COOCH_3$ | Cl | 58.14 | 3.43 | 5.02 | 58.16 | 3.62 | 4.96 | 210-212 |
| 36 | $C_{26}H_{16}Cl_2F_2N_2O_5$ | F | F | H | $CH_3$ | Cl | COOH | Cl | 57.27 | 2.96 | 5.14 | 56.87 | 3.05 | 4.91 | 235-238 |
| 37 | $C_{28}H_{20}Cl_2F_2N_2O_3S_2$ | F | F | H | $CH_3$ | Cl | $CH(SCH_2)_2$ | Cl | 55.54 | 3.33 | 4.63 | 54.66 | 3.36 | 4.79 | 93-98 |
| 38 | $C_{28}H_{21}Cl_3N_2O_3S_2$ | Cl | H | H | $CH_3$ | Cl | $CH(SCH_2)_2$ | Cl | 55.68 | 3.51 | 4.64 | 55.93 | 3.63 | 4.59 | 114-119 |

Certain representative examples of the new compounds were evaluated to determine their pesticidal activity against certain insects, including a caterpillar and a beetle.

Suspensions of the test compounds were prepared by dissolving 100 milligrams of compound in 1.5 milliliters of dimethylforamide and then adding 8.5 milliliters of an acetone solution containing 0.25 percent of an alkylphenoxy polyethoxyethanol surfactant, as an emulsifying or dispersing agent. The resulting solution was mixed into 30 milliliters of water to give roughly 40 milliliters of a suspension containing the compound in finely divided form. The thus-prepared stock suspension contained 2.5 percent by weight of compound. The test concentrations in parts per million by weight employed in the tests described hereinbelow were obtained by appropriate dilutions of the stock suspension with water. Sonication was used where necessary to obtain a homogeneous suspension. The test procedures were as follows:

Southern Armyworm Leaf Spray Test

Larvae of the southern armyworm (Spodoptera eridania, (Cram.)), reared on Tendergreen bean plants at a temperature of 80° ± 5° F. and a relative humidity of 50 ± 5 percent, constituted the test insects.

The test compounds were formulated by diluting the stock suspension with water to give a suspension containing the test compound at the concentrations (in parts of the test compound per million parts of final formulation) as set forth in the Tables below. Potted tendergreen bean plants of standard height and age were placed on a revolving turntable and sprayed with 100 milliliters of test compound formulation by use of a DeVilbiss spray gun set at 40 psig air pressure. This application, which lasted 25 seconds, was sufficient to wet plants to run-off. As a control, 100 milliliters of a water-acetone-emulsifier solution containing no test compound were also sprayed on infested plants. When dry, the paired leaves were separated and each one was placed in a 9 centimeter Petri dish lined with moistened filter paper. Five randomly selected larvae were introduced into each dish and the dishes were closed. The closed dishes were labeled and held at 80° -85° F. for five days. Although the larvae could easily consume the whole leaf within twenty-four hours, no more food was added. Larvae which were unable to move the length of the body, even upon stimulation by prodding, were considered dead. Percent mortality was recorded for various concentration levels.

Mexican Bean Beetle Leaf Spray Test

Third instar larvae of the Mexican bean beetle (Epilachna varivestis, Muls.), reared on Tendergreen bean plants at a temperature of 80° +_ 5° F. and 50±5 percent relative humidity, were the test insects.

The test compounds were formulated by diluting the stock suspension with water to give a suspension containing the test compound at the concentrations (in parts of the test compound per million parts of final formulation) as set forth in the Tables below. Potted Tendergreen bean plants of standard height and age were placed on a revolving turntable and sprayed with 100 milliliters of test compound formulation by use of a DeVilbiss spray gun set at 40 psig air pressure. This application, which lasted 25 seconds, was sufficient to wet plants to run-off. As a control, 100 milliliters of a water-acetone-emulsifier solution containing no test compound were also sprayed on infested plants. When dry, the paired leaves were separated and each was placed in a 9 centimeter Petri dish lined with moistened filter paper. Five randomly selected larvae were introduced into each dish, and the dishes were closed. The closed dishes were labeled and held at a temperature of 80°±5° F., for five days. Although the larvae could easily consume the leaf within 24 to 48 hours, no more food was added. Larvae which were unable to move the length of the body, even upon stimulation, were considered dead.

Tobacco Budworm and Cotton Bollworm Leaf Spray Bait Test

Second instar larvae of the tobacco budworm (weighing about 2.5 mg) (Heliothis virescens, F.) and the cotton bollworm (weighing about 2.5 mg) (Heliothis zea, (Boddie)), obtained commercially and reared on artificial diet at a temperature of 80°± 5° and a relative humidity of 50± 5 percent, constituted the test insects.

Using a procedure similar to the above, but substituting cotton plants for snapbeans, treated and dried cotton leaves were introduced into 9 cm Petri dishes which were organized in to groups of 10-dish sets. One randomly selected larvae was introduced into each dish of a ten dish set and the dishes were closed. The closed dishes were labelled and held at 80°± 5° F. for five days. Larvae which were unable to more the length of the body, even upon stimulation, were considered dead. Percent mortality was recorded for various concentration levels.

The biological properties of certain representative examples of the compounds of this invention are set forth in Tables III and IV below.

Table III
Biological Properties of Representative 1-(4-Aryloxyphenyl)-3-Benzoyl Urea Compounds

| Compound Prepared In Example No. | Activity at 100 ppm(3) SAW(1) | MBB(2) |
|---|---|---|
| 1 | A | A |
| 2 | A | C |
| 3 | A | A |
| 4 | A | C |
| 5 | A* | A* |
| 6 | A* | A* |
| 7 | A | A |
| 8 | A | A |
| 9 | A | A |
| 10 | A | A |
| 11 | A | A |
| 12 | A | A |
| 13 | A | A |
| 14 | A | A |
| 15 | A | A |
| 16 | A | A |
| 17 | A | A |
| 18 | A | B |
| 19 | A | A |
| 20 | A | A |
| 21 | A | A |
| 22 | A | A |
| 23 | A | A |
| 24 | A | A |
| 25 | A | A |
| 26 | A | A |
| 27 | A | A |
| 28 | A | A |
| 29 | A | A |
| 30 | A | A |
| 31 | A | A |
| 32 | A | A |
| 33 | A | A |
| 34 | A | A |
| 35 | A* | A* |
| 36 | A | A |
| 37 | A | A |
| 38 | B | C |

(1) Southern Armyworm
(2) Mexican Bean Beetle
(3) Code: A = 71 - 100% Kill
B = 31 - 70% Kill
C = 0 - 30% Kill

*Tested at 500 ppm.

Table IV
Biological Properties of Representative 1-(4-Aryloxyphenyl)-3-Benzoyl Urea Compounds

| Compound Prepared In Example No. | Activity at 31 ppm(1) H. Virescens | H. Zea |
|---|---|---|
| 1 | A | A |
| 6 | A | A |
| 8 | A | - |
| 11 | A | - |
| 21 | A | - |
| 23 | A | - |
| 28 | A | A |
| 29 | B | A |

(1) Code: A = 71 - 100% kill
B = 31 - 70% kill
C = 0 - 30% kill

Although the invention has been illustrated by the foregoing examples, it is not to be construed as being limited to the materials employed therein; but rather, the invention encompasses the generic area as hereinabove disclosed. Various modifications and embodiments can be made without departing from the spirit and scope thereof.

## Claims

1. A compound of the formula:

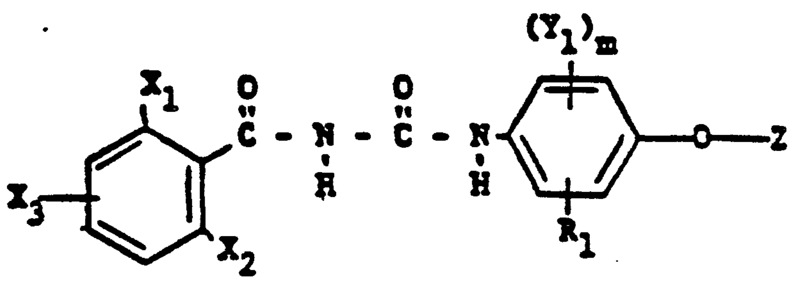

wherein:
$X_1$ represents halogen;
$X_2$ and $X_3$ independently represent hydrogen or halogen; .
$Y_1$ independently represents halogen, alkyl, alkoxy, nitro or cyano;
m is a value of from 0 to 2;
$R_1$ represents an acyclic or cyclic acetal group, an acyclic or cyclic dithioacetal group, an acyclic or cyclic hemithioacetal group, aldehyde group, carboxylic acid group or ester derivative thereof, hydroxyalkyl group, alkoxyalkyl group, acyloxyalkyl group, alkenyl group or alkylcarbonyl group; with the proviso that when $R_1$ is

a carboxylic acid group or ester derivative thereof, then m is a value of 2, and with the further proviso that $R_1$ is situated at other than the 2-or 6-position; and
Z represents (i) a substituted or unsubstituted, saturated or unsaturated monocyclic ring system or (ii) a substituted or unsubstituted, saturated or unsaturated bicyclic fused ring system wherein at least one ring is a six-membered unsaturated carbocyclic ring and the second ring is a five-or six-membered carbocyclic ring or a five-or six-membered heterocyclic ring which can contain in any combination a carbonyl group or one or two oxygen or sulfur atoms.

2. A compound as claimed in Claim 1 which has the formula:

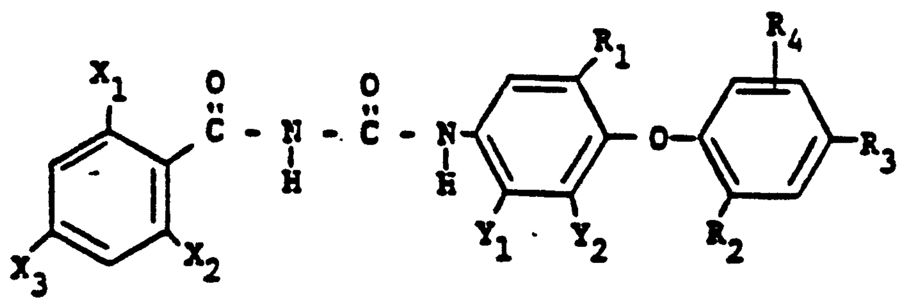

wherein $X_1$, $X_2$, $X_3$, $R_1$ and $Y_1$ are as defined in claim 1, $Y_2$ independently has the meaning given for $Y_1$, and $R_2$, $R_3$ and $R_4$ are independently hydrogen, halogen, alkyl, haloalkyl of polyhaloalkyl.

3. A compound as claimed in Claim 1, which has the formula:

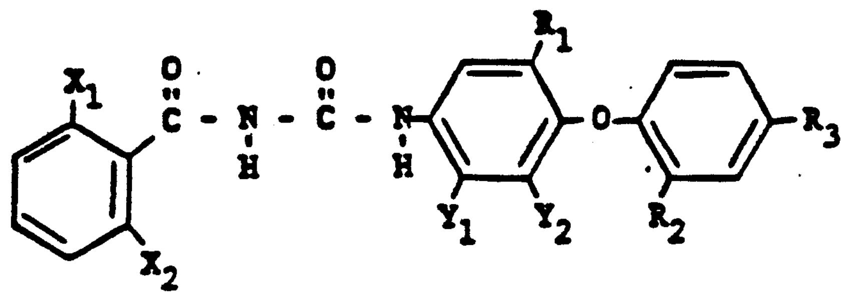

wherein $X_1$, $X_2$ and $R_1$ are as defined in claim 1, $Y_2$ independently has the meaning given for $Y_1$, and $R_2$ and $R_3$ are independently hydrogen, halogen, alkyl, haloalkyl or polyhaloalkyl.

4. A compound as claimed in claim 1 which has the formula:

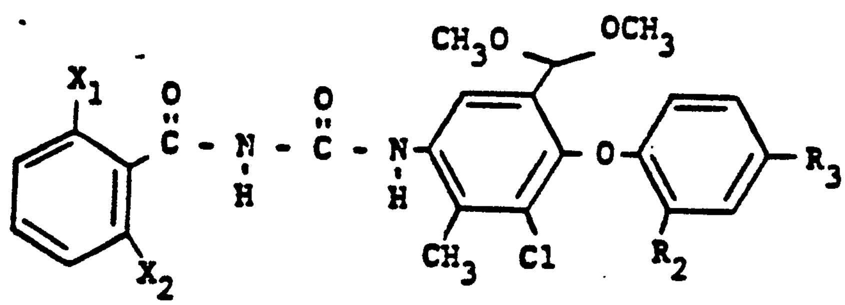

wherein $X_1$ and $X_2$ are as defined in claim 1, and $R_2$ and $R_3$ are independently hydrogen, halogen, alkyl, haloalkyl or polyhaloalkyl.

5. A compound as claimed in claim 1 which has the formula:

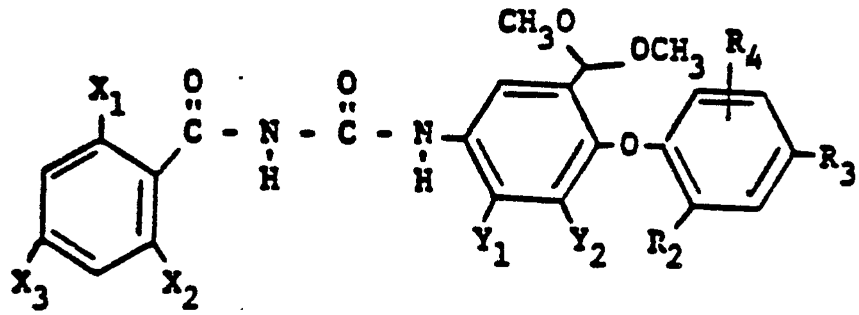

wherein $X_1$, $X_2$, $X_3$ and $Y_1$ are as defined in claim 1, $Y_2$ independently has the meaning given for $Y_1$, and $R_2$, $R_3$ and $R_4$ are independently hydrogen, halogen, alkyl, haloalkyl or polyhaloalkyl.

6. A compound as claimed in claim 1 which has the formula:

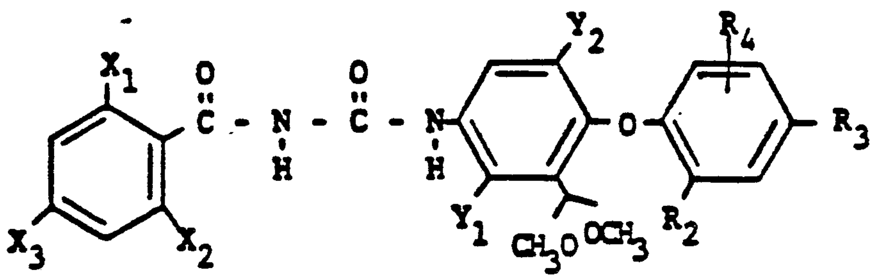

wherein $X_1$, $X_2$, $X_3$ and $Y_1$ are as defined in claim 1, $Y_2$ independently has the meaning given for $Y_1$, and $R_2$, $R_3$ and $R_4$ are independently hydrogen, halogen, alkyl, haloalkyl or polyhaloalkyl.

7. A compound as claimed in claim 1 which has the formula:

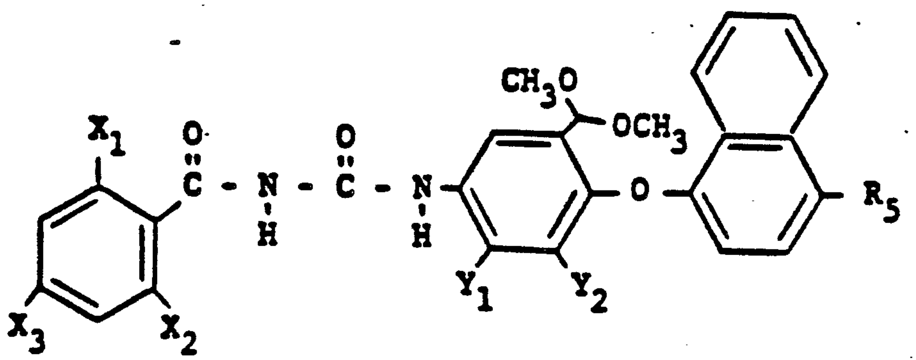

wherein $X_1$, $X_2$, $X_3$ and $Y_1$ are as defined in claim 1, $Y_2$ independently has the meaning given for $Y_1$, and $R_5$ is hydrogen, halogen, alkyl, haloalkyl or polyhaloalkyl.

8. A compound as claimed in claim 1 which has the formula:

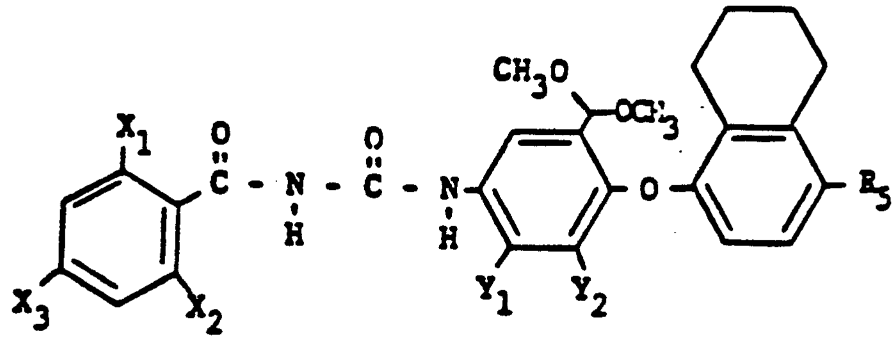

wherein $X_1$, $X_2$, $X_3$ and $Y_1$ are as defined in claim 1, $Y_2$ independently has the meaning given for $Y_1$, and $R_5$ is hydrogen, halogen, alkyl, haloaklyl or polyhaloalkyl.

9. A compound as claimed in claim 1 which has the formula:

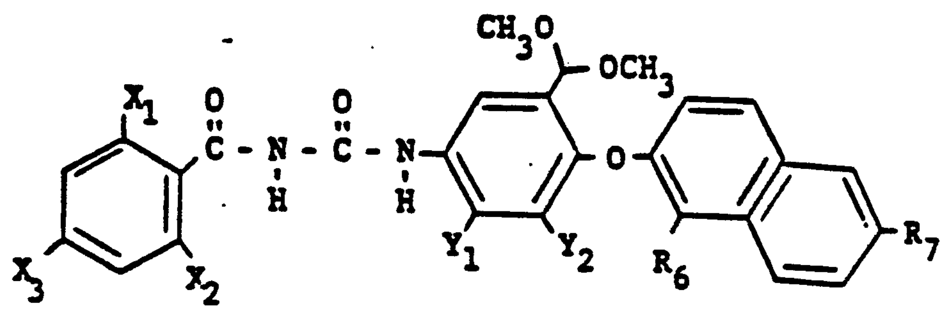

wherein $X_1$, $X_2$, $X_3$ and $Y_1$ are as defined in claim 1, $Y_2$ independently has the meaning given for $Y_1$, and $R_6$ and $R_7$ are independently hydrogen, halogen, alkyl, haloalkyl or polyhaloalkyl.

10. A compound as claimed in claim 1 which has the formula:

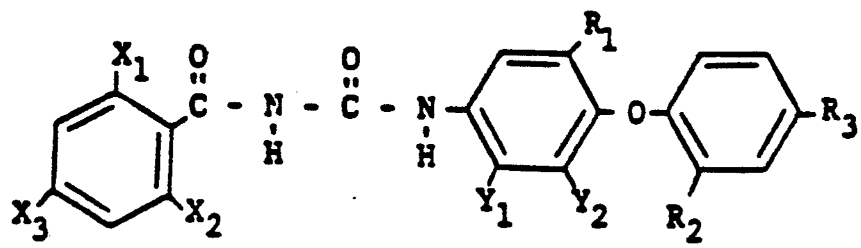

wherein $X_1$, $X_2$, $X_3$, and $Y_1$ and $R_1$ are as defined in claim 1, $Y_2$ independently has the meaning given for $Y_1$, and $R_2$, and $R_3$ are independently hydrogen, halogen, alkyl, haloalkyl or polyhaloalkyl.

11. A compound as claimed in claim 1 which has the formula:

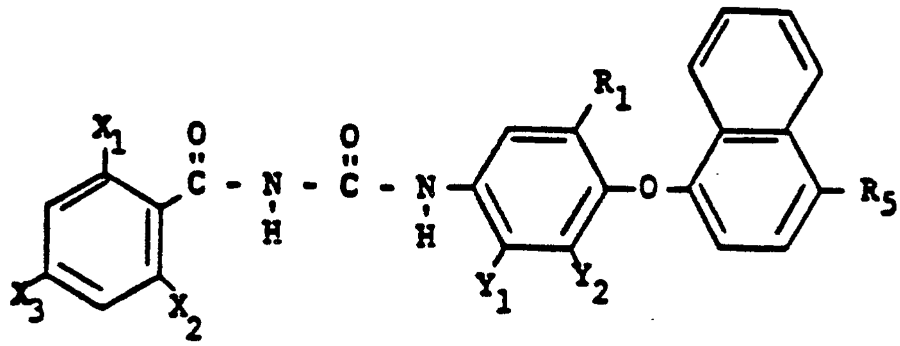

wherein $X_1$, $X_2$, $X_3$, $Y_1$ and $R_1$ are as defined in claim 1, $Y_2$ independently has the meaning given for $Y_1$, and $R_5$ and is hydrogen, halogen, alkyl, haloalkyl or polyhaloalkyl.

12. A compound as claimed in claim 1 which has the formula:

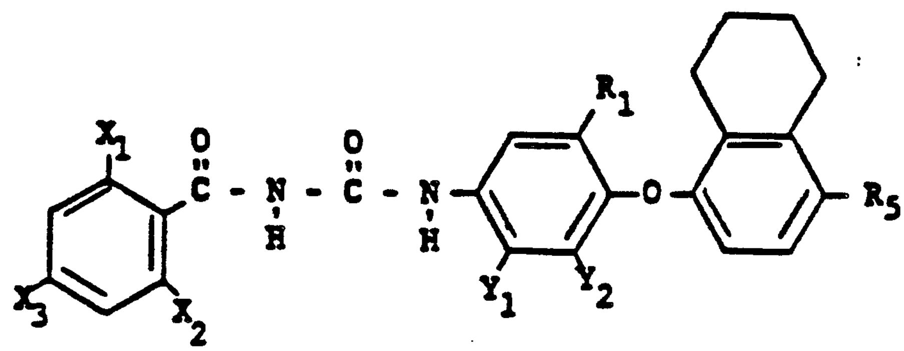

wherein $X_1$, $X_2$, $X_3$, $R_1$ and $Y_1$ are as indicated in claim 1, $Y_2$ independently has the meaning given for $Y_1$, and $R_5$ is hydrogen, alkyl or halogen.

13. A compound as claimed in claim 1 which has the formula:

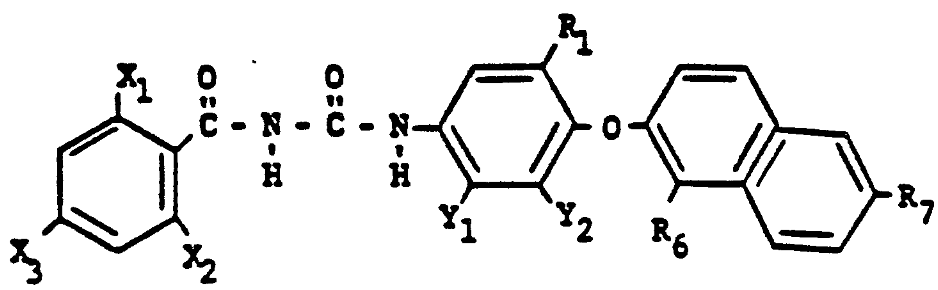

wherein $X_1$, $X_2$, $X_3$, $R_1$ and $Y_1$ are as defined in claim 1, $Y_2$, independently has the meaning given for $Y_1$, and $R_6$ and $R_7$ are independently hydrogen; alkyl or halogen.

14. A compound as claimed in claim 1 wherein Z is selected from a substituted or unsubstituted ring system having the formula:

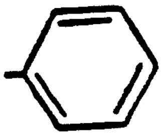 , 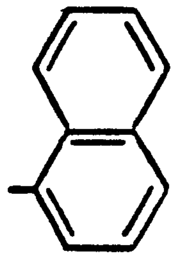 , 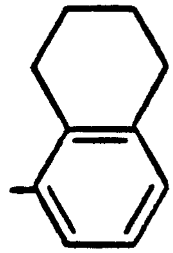 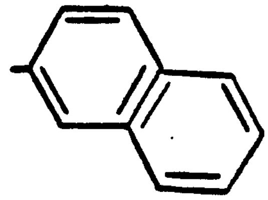

wherein the permissible substituents are one or more halogen, alkyl, haloalkyl, polyhaloalkyl, nitro or cyano in any combination.

15. A compound as claimed in claim 1 wherein $R_1$ is one of the following groups:-

(i) an acyclic acetal group having the formula:

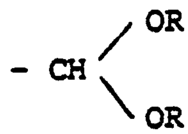

wherein each R is independently alkyl, alkenyl, alkynyl, polyhaloalkyl or polyhaloalkenyl;

(ii) an acyclic dithioacetal group having the formula:

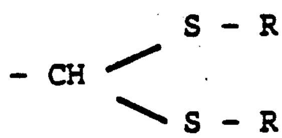

wherein each R is independently alklyl, alkenyl, alkynyl, polyhaloalkyl or polyhaloalkenyl;

(iii) a cyclic acetal group having the formula:

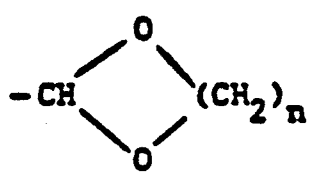

wherein n is a value of 2 or 3;

(iv) a cyclic dithioacetal group having the formula:

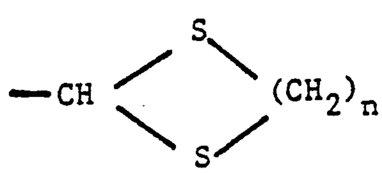

wherein n is a value of 2 or 3;

(v) a cyclic hemithioacetal group having the formula:

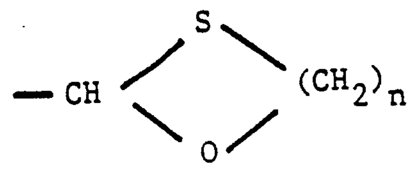

wherein n is a value of 2 or 3;

(vi) a carboxylic acid ester group having the formula:

$-CO_2R$

wherein R is alkyl, alkenyl, alkynyl, polyhaloalkyl or polyhaloalkenyl;

(vii) an acyloxyalkyl group having the formula:

$-CH_2OCOR$

wherein R is alkyl, alkenyl, alkynyl, polyhaloalkyl or polyhaloalkenyl;

(viii) an alkoxyalkyl group having the formula:

$-CH_2OR$

wherein R is alkyl, alkenyl, alkynyl, polyhaloalkyl or polyhaloalkenyl; or

(ix) an alkenyl group having the formula:

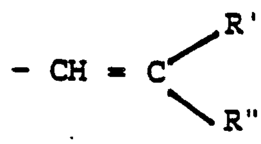

wherein R' and R" are independently hydrogen, halogen, nitro, cyano or an ester group.

16. A compound as claimed in claim 1 which is one of the following compounds:-
1-[3-chloro-4-(2,4-dichlorophenoxy)-5-dimethoxymethyl-2-methylphenyl]-3-(2,6-difluorobenzoyl)urea;
1-[3-chloro-4-(2,4-dichlorophenoxy)-5-formyl-2-methylphenyl]-3-(2,6-difluorobenzoyl)urea;
1-[3-chloro-4-(2,4-dichlorophenoxy)-5-hydroxymethyl-2-methylphenyl]-3-(2,6-difluorobenzoyl)urea;
1-[5-carboxy-3-chloro-4-(2,4-dimethylphenoxy)-2-methylphenyl]-3-(2,6-difluorobenzoyl)urea;
1-[3-chloro-4-(2,4-dimethylphenoxy)-5-(2-methoxy-carbonyl-1-ethenyl)-2-methylphenyl]-3-(2,6-difluorobenzoyl)urea;
1-[3-chloro-4-(2,4-dichlorophenoxy)-5-dimethoxymethyl-2-methylphenyl]-3-(2,6-dichlorobenzoyl)urea;
1-[3-chloro-4-(2,4-dimethylphenoxy)-5-dimethoxymethyl-2-methylphenyl]-3-(2,6-difluorobenzoyl)urea;
1-[3-chloro-4-(2,4-dimethylphenoxy)-5-dimethoxymethyl-2-methylphenyl]-3-(2,6-dichlorobenzoyl)urea;
1-[3-chloro-4-(2-bromo-4-chlorophenoxy)-5-dimethoxymethyl-2-methylphenyl]-3-(2,6-difluorobenzoyl)urea;
1-[3-chloro-4-(2-bromo-4-chlorophenoxy)-5-dimethoxymethyl-2-methylphenyl]-3-(2,6-dichlorobenzoyl)urea;
1-[3-chloro-4-(4-chloronaphthoxy)-5-dimethoxymethyl-2-methylphenyl]-3-(2,6-difluorobenzoyl)urea;
1-[3-chloro-4-(4-chloronaphthoxy)-5-dimethoxymethyl-2-methylphenyl]-3-(2,6-dichlorobenzoyl)urea;
1-(3-chloro-4-(2,4-dichlorophenoxy)-5-(1,3-dithiacyclopentan-2-yl)-2-methylphenyl)-3-(2,6-difluorobenzoyl)-urea;
1-(3-chloro-4-(4-chloronaphthoxy)-5-(1,3-dithiacyclopentan-2-yl)-2-methylphenyl)-3-(2,6-difluorobenzoyl)urea;
1-(3-chloro-4-(2,4-difluorophenoxy)-5-(1,3-dithiacyclopentan-2-yl)-2-methyphenyl)-3-(2,6-difluorobenzoyl)-urea; or
1-(3-chloro-4-(2,4-dichlorophenoxy)-2-methyl-5-(3-oxa-1-thiacyclopentan-2-yl)phenyl)-3-(2,6-difluorobenzoyl)-urea.

17. A pesticide composition comprising an acceptable carrier and a pesticidally effective amount of the compound claimed in any one of claims 1 to 16.

18. A method of controlling pests which comprises subjecting said pests to a pesticidally effective amount of a compound as claimed in any one claims 1 to 16.

19. A process for the preparation of a compound as claimed in claim 1 which process comprises reacting a aryloxyaniline of the formula:

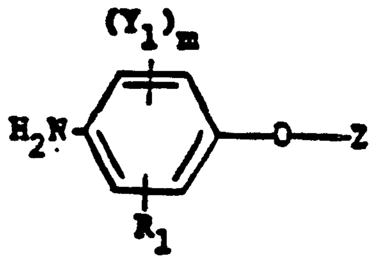

with a benzoyl isocyanate of the formula:

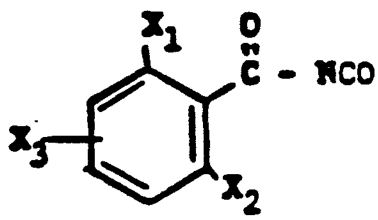

wherein:
$X_1$ represents halogen;
$X_2$ and $X_3$ independently represent hydrogen or halogen;
$Y_1$ independently represents halogen, alkyl, alkoxy, nitro or cyano;
m is a value of from 0 to 2;
$R_1$ represents an acyclic or cyclic acetal group, an acyclic or cyclic dithioacetal group, an acyclic or cyclic hemithioacetal group, aldehyde group, carboxylic acid group or ester derivative thereof, hydroxyalkyl group, alkoxyalkyl group, acyloxyalkyl group, alkenyl group or alkylcarbonyl group; with the proviso that when $R_1$ is a carboxylic acid group or ester derivative thereof, then m is a value of 2, and with the further proviso that $R_1$ is situated at other than the 2-or 6-position; and
Z represents (i) a substituted or unsubstituted, saturated or unsaturated monocyclic ring system or (ii) a substituted or unsubstituted, saturated or unsaturated bicyclic fused ring system wherein at least one ring is a six-membered unsaturated carbocyclic ring and the second ring is a five-or six-membered carbocyclic ring or a five-or six-membered heterocyclic ring which can contain in any combination a carbonyl group or one or two oxygen or sulfur atoms.

20. A process for the preparation of a compound as claimed in claim 1 which process comprises reacting a aryloxyphenylisocyanate of the formula:

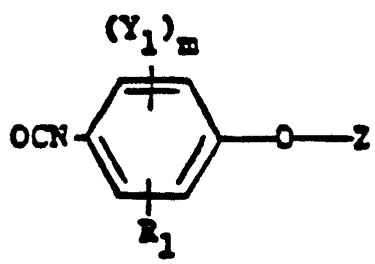

with a benzamide of the formula:

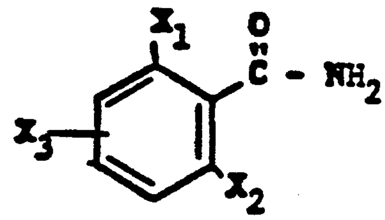

wherein:

$X_1$ represents halogen;

$X_2$ and $X_3$ independently represent hydrogen or halogen;

$Y_1$ independently represents halogen, alkyl, alkoxy, nitro or cyano;

m is a value of from 0 to 2;

$R_1$ represents an acyclic or cyclic acetal group, an acyclic or cyclic dithioacetal group, an acyclic or cyclic hemithioacetal group, aldehyde group, carboxylic ester group, alkoxyalkyl group, acyloxyalkyl group, alkenyl group or alkylcarbonyl group; with the proviso that when $R_1$ is a carboxylic ester group, then m is a value of 2, and with the further proviso that $R_1$ is situated at other than the 2-or 6-position; and

Z represents (i) a substituted or unsubstituted, saturated or unsaturated monocyclic ring system or (ii) a substituted or unsubstituted, saturated or unsaturated bicyclic fused ring system wherein at least one ring is a six-membered unsaturated carbocyclic ring and the second ring is a five-or six-membered carbocyclic

21. A process for the preparation of a compound as claimed in claim 1 which process comprises reacting s substituted urea of the formula:

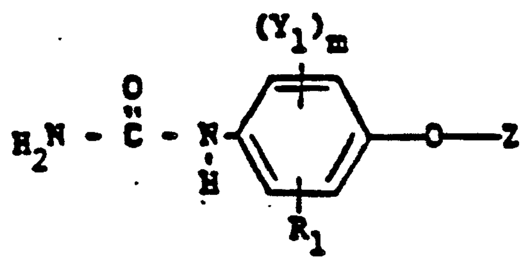

with a benzoyl chloride of the formula:

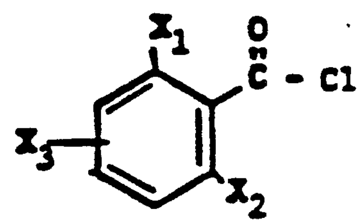

wherein:

$X_1$ represents halogen;

$X_2$ and $X_3$ independently represent hydrogen or halogen;

$Y_1$ independently represents halogen, alkyl, alkoxy, nitro or cyano;

m is a value of from 0 to 2;

$R_1$ represents a carboxylic ester group, alkoxyalkyl group, acyloxyalkyl group, alkenyl group or alkylcarbonyl group; with the proviso that when $R_1$ is a carboxylic ester group, then m is a value of 2, and with the further proviso that $R_1$ is situated at other than the 2-or 6-position; and

Z represents (i) a substituted or unsubstituted, saturated or unsaturated monocyclic ring system or (ii) a substituted or unsubstituted, saturated or unsaturated bicyclic fused ring system wherein at least one ring is a six-membered unsaturated carbocyclic ring and the second ring is a five-or six-membered carbocyclic ring or a five-or six-membered heterocyclic ring which can contain in any combination a carbonyl group or one or two oxygen or sulfur atoms.